(19) **Europäisches Patentamt / European Patent Office / Office européen des brevets**

(11) **EP 3 496 732 B1**

(12) # EUROPEAN PATENT SPECIFICATION

(45) Date of publication and mention
of the grant of the patent:
**15.07.2026   Bulletin 2026/29**

(21) Application number: **17840318.4**

(22) Date of filing: **11.08.2017**

(51) International Patent Classification (IPC):
*A61K 31/78* (2006.01)          *A61K 35/42* (2015.01)
*A61K 31/765* (2006.01)         *A61K 47/34* (2017.01)
*A61K 45/06* (2006.01)          *A61K 31/787* (2006.01)
*A61K 31/77* (2006.01)          *A61K 31/74* (2006.01)
*A61K 9/107* (2006.01)          *A61K 9/00* (2006.01)
*A61P 11/00* (2006.01)          *A61K 31/715* (2006.01)

(52) Cooperative Patent Classification (CPC):
(C-Sets available)
**A61P 11/00; A61K 9/0082; A61K 9/1075;
A61K 31/74; A61K 31/765; A61K 31/77;
A61K 31/78; A61K 31/787; A61K 45/06;
A61K 47/34**                                    (Cont.)

(86) International application number:
**PCT/US2017/046426**

(87) International publication number:
**WO 2018/031850 (15.02.2018 Gazette 2018/07)**

(54) **POLYMER LUNG SURFACTANTS**

POLYMERLUNGENTENSIDE

POLYMÈRES TENSIOACTIFS PULMONAIRES.

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **12.08.2016   US 201662374325 P**

(43) Date of publication of application:
**19.06.2019   Bulletin 2019/25**

(73) Proprietor: **Purdue Research Foundation
West Lafayette, IN 47906 (US)**

(72) Inventors:
• **WON, You-Yeon
West Lafayette, Indiana 47906 (US)**
• **KIM, Hyun Chang
Wilmington, DE 19806 (US)**

(74) Representative: **Secerna LLP
The Old Fire Station
18 Clifford Street
York YO1 9RD (GB)**

(56) References cited:
EP-A1- 2 918 264          WO-A1-2010/046681
WO-A1-2013/106100         WO-A1-97/10849
WO-A1-97/10849            CN-A- 101 804 021
US-A1- 2013 004 453       US-A1- 2013 004 453
US-A1- 2013 228 181       US-B1- 6 180 142
US-B2- 6 939 564          US-B2- 8 529 917

• HAE-WOONG PARK ET AL: "Study of the Air-
Water Interfacial Properties of Biodegradable
Polyesters and Their Block Copolymers with
Poly(ethylene glycol)", LANGMUIR, vol. 28, no.
31, 7 August 2012 (2012-08-07), US, pages 11555 -
11566, XP055676450, ISSN: 0743-7463, DOI:
10.1021/la300810q
• HYUN CHANG KIM ET AL: "-lactic acid- ran
-glycolic acid- ran -[epsilon]-caprolactone)-
block -ethylene glycol) (PLGACL-PEG) Block
Copolymers at the Air-Water Interface",
LANGMUIR, vol. 31, no. 51, 15 December 2015
(2015-12-15), US, pages 13821 - 13833,
XP055676057, ISSN: 0743-7463, DOI: 10.1021/
acs.langmuir.5b03622

EP 3 496 732 B1

- JULIET K. COX ET AL: "Polystyrene-Poly(ethylene oxide) Diblock Copolymers Form Well-Defined Surface Aggregates at the Air/Water Interface", LANGMUIR, vol. 15, no. 22, 1 October 1999 (1999-10-01), US, pages 7714 - 7718, XP055676771, ISSN: 0743-7463, DOI: 10.1021/la9901940
- A. MALZERT ET AL: "Interfacial Properties of a PEG2000-PLA50 Diblock Copolymer at the Air/Water Interface", LANGMUIR, vol. 17, no. 25, 1 December 2001 (2001-12-01), US, pages 7837 - 7841, XP055676762, ISSN: 0743-7463, DOI: 10.1021/la010818d
- MAXIME RANGER ET AL: "From well-defined diblock copolymers prepared by a versatile atom transfer radical polymerization method to supramolecular assemblies", JOURNAL OF POLYMER SCIENCE, PART A: POLYMER CHEMISTRY, JOHN WILEY & SONS, INC, US, vol. 39, no. 22, 15 November 2001 (2001-11-15), pages 3861 - 3874, XP009136940, ISSN: 0887-624X, [retrieved on 20011002], DOI: 10.1002/POLA.10029
- HYUN CHANG KIM ET AL: "Polymer Lung Surfactants", ACS APPLIED BIO MATERIALS, vol. 1, no. 3, 22 August 2018 (2018-08-22), pages 581 - 592, XP055676448, ISSN: 2576-6422, DOI: 10.1021/acsabm.8b00061
- HYUN CHANG KIM ET AL: "-lactic acid- co -glycolic acid)- block -ethylene glycol) (PLGA-PEG) Polymers", LANGMUIR, vol. 34, no. 16, 24 April 2018 (2018-04-24), US, pages 4874 - 4887, XP055676452, ISSN: 0743-7463, DOI: 10.1021/acs.langmuir.8b00566
- FESENMEIER DANIEL J. ET AL: "Surface mechanical behavior of water-spread poly(styrene)-poly(ethylene glycol) (PS-PEG) micelles at the air-water interface: Effect of micelle size and polymer end/linking group chemistry", JOURNAL OF COLLOID AND INTERFACE SCIENCE, vol. 617, 1 July 2022 (2022-07-01), US, pages 764 - 777, XP093077785, ISSN: 0021-9797, DOI: 10.1016/j.jcis.2022.03.008
- HO-CHUL SHIN ET AL: "A 3-in-1 Polymeric Micelle Nanocontainer for Poorly Water-Soluble Drugs", MOLECULAR PHARMACEUTICS, vol. 8, no. 4, 25 August 2011 (2011-08-25), US, pages 1257 - 1265, XP055649865, ISSN: 1543-8384, DOI: 10.1021/mp2000549
- MENON JYOTHI U. ET AL: "Polymeric nanoparticles for pulmonary protein and DNA delivery", ACTA BIOMATERIALIA, vol. 10, no. 6, 1 June 2014 (2014-06-01), AMSTERDAM, NL, pages 2643 - 2652, XP093078551, ISSN: 1742-7061, Retrieved from the Internet <URL:https://www.sciencedirect.com/science/article/pii/S174270611400052X/pdfft?md5=62800f0be1419c15a5dc9910efae75a5&pid=1-s2.0-S174270611400052X-main.pdf> DOI: 10.1016/j.actbio.2014.01.033
- HENNING ANDREAS ET AL: "Influence of Particle Size and Material Properties on Mucociliary Clearance from the Airways", JOURNAL OF AEROSOL MEDICINE AND PULMONARY DRUG DELIVERY, vol. 23, no. 4, 1 August 2010 (2010-08-01), US, pages 233 - 241, XP093078554, ISSN: 1941-2711, DOI: 10.1089/jamp.2009.0806

(52) Cooperative Patent Classification (CPC): (Cont.)

C-Sets
A61K 31/74, A61K 2300/00;
A61K 31/765, A61K 2300/00;
A61K 31/77, A61K 2300/00;
A61K 31/78, A61K 2300/00;
A61K 31/787, A61K 2300/00

**Description**

**TECHNICAL FIELD**

The present disclosure generally relates to polymer lung surfactants and in particular to polymer lung surfactant materials that satisfy all surfactant performance requirements and have better handling characteristics than current respiratory distress syndrome therapeutics.

**BACKGROUND**

This section introduces aspects that may help facilitate a better understanding of the disclosure. Accordingly, these statements are to be read in this light and are not to be understood as admissions about what is or is not prior art.

Infants who are born before the full 40-week gestation period are considered "preterm" (if born before week 37 of pregnancy) or "premature" (if born before 34 week). One of the major health risks associated with preterm/premature births is underdeveloped lungs, which cause high infant mortality. Infants born before the 37th week gestation are born without alveolar structures, and have low production of lung surfactants. As a result, preterm/premature infants struggle to breathe, and, without proper treatments, die within a few days. This respiratory failure is named as Respiratory Distress Syndrome (RDS), or also known as Hyaline Membrane Disease, misnamed in the past due to the misconception of the cause of this disease as being of viral origin.

In old days when RDS was misnamed as Hyaline Membrane Disease, it was the leading cause of infant death in the United States with a higher death rate than Pneumonia and Influenza. However, now-a-days with skilled physicians and three well-established treatment methods, the mortality rate from RDS decreased substantially. The three treatments are performed in stages where if the earlier treatment is successful the next treatment is not performed. The first treatment for RDS is a prevention treatment where steroid is given to the mother 24 hours prior to labor to increase the production of the infant's own lung surfactants. Clinical data on steroid treatment with betamethasone has shown effective reduction of RDS occurrence from 25.8% to 9 %. The second treatment, Surfactant Replacement Therapy (SRT), involves intratracheal injection of animal extracted lung surfactants into the infant's lungs immediately after birth. The development of successful SRT has been the main driver in lowering the RDS-related mortality rate, and, due to its high effectiveness, is included in the Essential Drug List of the World Health Organization. The third treatment involves mechanical ventilation in which infants are put under nasal continuous/discontinuous positive airway pressure treatment to increase the oxygen levels in the lungs. Treatment using mechanical ventilation is the oldest treatment method for treating RDS. Its initial clinical testing was shown to reduce the mortality rate from 80 % to 20 %. However, oxygen poisoning and mechanical damage to the lungs remain an adverse effect. Out of the three treatment methods, SRT is the most reliable treatment directly resolving the underlying cause of RDS with no adverse effect reported so far. Improvement of RDS treatment is expected to be with advancements in SRT practice. US2013/004453A1 discloses synthetic nylon-3 co-polymers and synthetic lung surfactant compositions containing the same. Park et al (Langmuir 2012, 28, 31, 11555-11566) discloses a study of the air-water interfacial properties of biodegradable polyesters and their block copolymers with poly(ethylene glycol). Kim et al (Langmuir 2015, 31, 51, 13821-13833) discloses surface mechanical and rheological behaviors of biocompatible poly((d,l-lactic acid-ran-glycolic acid)-block-ethylene glycol) (PLGA-PEG) and poly((d,l-lactic acid-ran-glycolic acid-ran-ε-caprolactone)-block-ethylene glycol) (PLGACL-PEG) block copolymers at the air-water interface. Cox et al (Langmuir 1999, 15, 22, 7714-7718) discloses polystyrene-poly(ethylene oxide) diblock copolymers that form well-defined surface aggregates at the air/water interface. Malzert et al (Langmuir 2001, 17, 25, 7837-7841) discloses interfacial properties of a PEG2000-PLA50 diblock copolymer at the air/water interface. Ranger et al (Journal of Polymer Science Part A: Polymer Chemistry 2001, 39, 22, 3861-3874) discloses well-defined diblock copolymers prepared by a versatile atom transfer radical polymerization method to supramolecular assemblies. Shin et al (Mol. Pharmaceutics 2011, 8, 4, 1257-1265) discloses a polymeric micelle nanocontainer for poorly water-soluble drugs. CN101804021A discloses a preparation method for a polyene-containing taxol nanoparticle mixed micelle preparation and freeze-drying agent. WO97/10849A1 discloses a miscellar drug delivery system comprising a block copolymer having both hydrophobic and hydrophilic blocks. US8529917B2 discloses micelle encapsulation of a combination of therapeutic agents. Menon et al (Acta Biomaterialia 2014, 10, 6, 2643-2652) discloses polymeric nanoparticles for pulmonary protein and DNA delivery. Henning et al (Journal of Aerosol Medicine and Pulmonary Drug Delivery 2010, 23, 4, 233-241) discloses a study of the influence of particle size and material properties on mucociliary clearance from the airways.

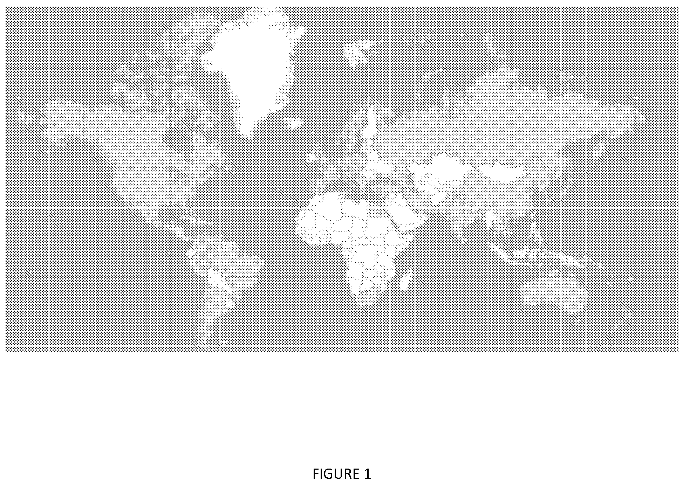

Despite the success in the domestic reduction of the RDS-related mortality for preterm and premature infants, world-wide, especially in developing countries, RDS is currently still one of the leading causes of neonatal death due to the high treatment cost and complex treatment procedures. Although highly effective, the cost of SRT is extremely high; the cost of SRT therapeutics alone exceeds the per capita GNP in some countries. The economical imbalance affecting the use of SRT is clearly show in Figure 1 where countries in Central Asia and Africa do not have full access to SRT. Development of lower-cost RDS therapeutics with simpler treatment procedures that do not require highly skilled physicians and advanced neonatal intensive care units (NICU) will solve this problem, and will reduce the leading cause of neonatal death world-wide. It should be noted that even in the United States, in some rural areas, preterm and premature infants are exposed to risks of RDS-related mortality due to the lack of skilled physicians and needed medical resources such as NICU facilities. In regions where SRT is not practicable, treatment mainly relies on mechanical ventilation. There is therefore an unmet need for a better SRT technology.

## BRIEF DESCRIPTION OF THE FIGURES

**Figure 1.** Countries in which Surfactant Replacement Therapy (SRT) is currently being practiced are colored in green. Countries were marked as "SRT practicing" if patients have access to at least one marketed lung surfactant therapeutic. Data were collected using Medtrack on March 24th 2015.

**Figure 2.** The Young-Laplace pressure of a spherical alveolus is calculated as a function of surface tension and radius.

**Figure 3.** Surface tension-relative area isotherms of a commercial lung surfactant, Survanta (AbbVie), obtained during repeated compression-expansion cycles. The subphase solution used contained 150 mM NaCl, 2 mM $CaCl_2$ and 0.2 mM $NaHCO_3$ (pH 7.0 - 7.4, 25 °C). The monolayer was compressed/expanded at a rate of 50 mm/min; one compression-expansion cycle took 7.18 minutes. The data displayed represent the last 10 compression-expansion cycles of a total 50 continuous cycles performed after spreading 4 mg Survanta.

**Figure 4.** Chemical structures of PEG-based block copolymers tested for lung surfactant applications.

**Table 1.** Molecular characteristics of polymer lung surfactant candidate materials investigated. [1] lactide:glycolide = 70:55 by mole. [2] lactide:glycolide:caprolactone = 30:29:41 by mole.

| Polymer | Mn,PEG (g/mol) | $M_{n,PLGA/PLGACL/PS/PtBMA}$ (g/mol) | PDI |
|---|---|---|---|
| PLGA-PEG | 5,000 | 8,333[1] | 1.46 |
| PLGACL-PEG | 5,000 | 9,857[2] | 1.33 |
| PtBMA-PEG | 5,000 | 1,785 | 1.12 |
| PS(1560)-PEG(5000) | 5,000 | 1,560 | 1.10 |
| PS(2993)-PEG(5000) | 5,000 | 2,993 | 1.13 |
| PS(5610)-PEG(5000) | 5,000 | 5,610 | 1.16 |
| PS(13832)-PEG(5000) | 5,000 | 13,832 | 1.38 |

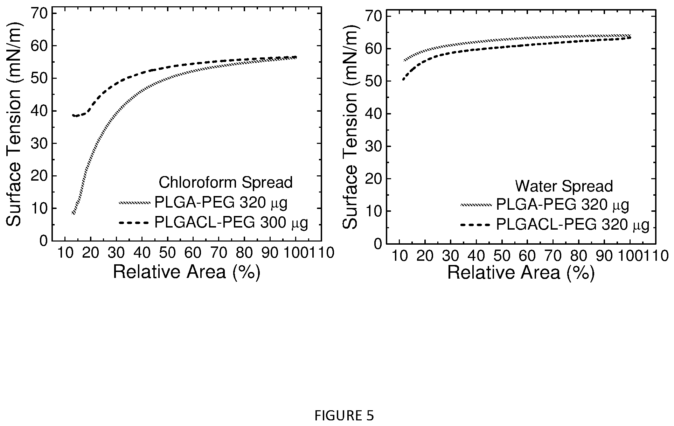

**Figure 5.** Constant-compression surface tension-area isotherms of (a) chloroform-spread and (b) water-spread PLGA-PEG and PLGACL-PEG monolayers on the surface of Millio-Q-purified water (18 MΩ·cm resistivity) at 25 °C. Surface tension was measured during compression at a rate of 3 mm/min. The hydrodynamic diameters of the PLGA-PEG and PLGACL-PEG micelles were 144.1 and 53.0 nm, respectively (measured by DLS).

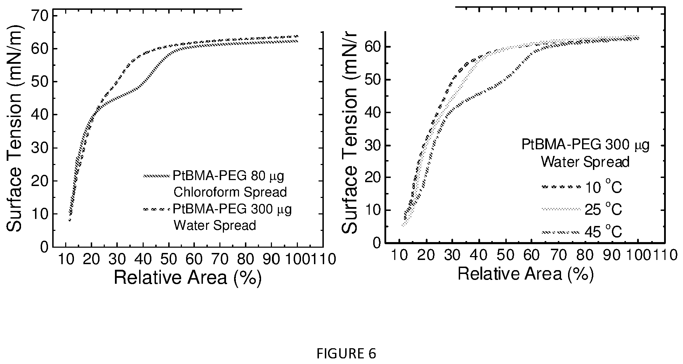

Figure 6**.** (a) Constant-compression surface tension-area isotherms of chloroform-spread and water-spread PtBMA-PEG monolayers at 25 °C. (b) Constant-compression surface tension-area isotherms of water-spread PtBMA-PEG monolayers at three different temperatures, 10, 25 and 40 °C. In all measurements Milli-Q-purified water (18 MΩ·cm resistivity) was used as the subphase, and the monolayer compression rate was 3 mm/min. The hydrodynamic diameter of the PtBMA-PEG micelles was 26.3 ± 3.9 nm (measured by DLS).

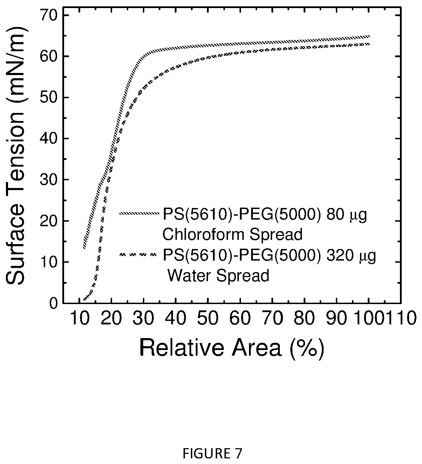

**Figure 7.** Surface tension-area isotherms of chloroform-spread versus water-spread PS-PEG monolayers under continuous compression at a rate of 3 mm/min on Milli-Q-purified water (18 MΩ·cm resistivity) at 25 °C. The numbers within the parentheses represent the number-average molecular weights of the respective blocks (g/mol).

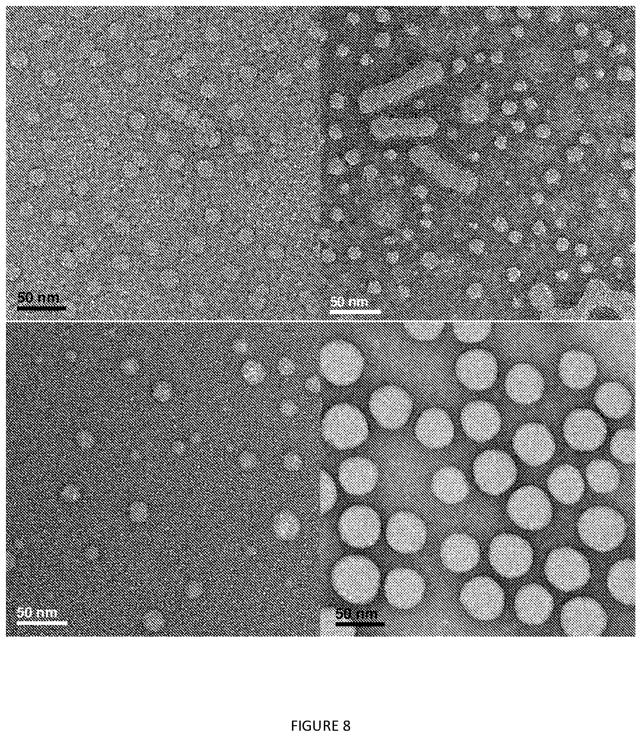

**Figure 8.** TEM images of (a) PS(1560)-PEG(5000), (b) PS(2993)-PEG(5000), (c) PS(5610)-PEG(5000) and (d) PS(13832)-PEG(5000) micelles formed in bulk water solutions. The dried micelle samples were negatively stained with uranyl acetate.

**Table 2.** Diameters of PS-PEG micelles as determined by TEM (Figure 8) or DLS.

| Block Copolymer | Micelle Diameter Determined by TEM | Micelle Diameter Determined by DLS |
|---|---|---|
| PS(1560)-PEG(5000) | 14.5 ± 1.7 nm (excluding elongated micelles) | 34.5 ± 2.0 nm |
| PS(2993)-PEG(5000) | 16.00 ± 4.7 nm | 23.2 ± 2.0 nm |
| PS(5610)-PEG(5000) | 20.0 ± 1.7 nm | 27.3 ± 0.2 nm |

(continued)

| Block Copolymer | Micelle Diameter Determined by TEM | Micelle Diameter Determined by DLS |
|---|---|---|
| PS(13832)-PEG(5000) | $45.3 \pm 2.7$ nm | $55.8 \pm 0.3$ nm |

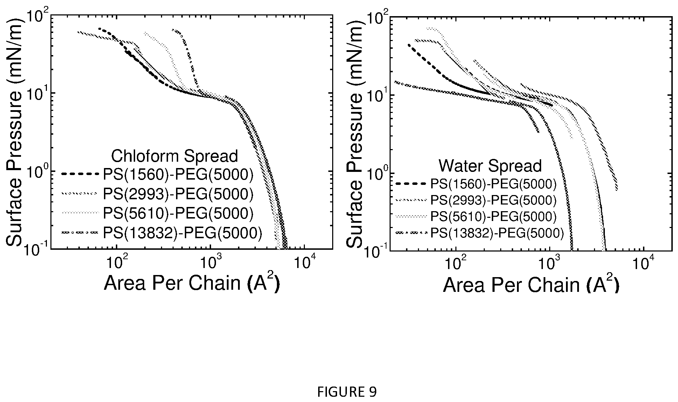

**Figure 9.** Constant-compression surface pressure-area isotherms of (a) chloroform-spread and (b) water-spread monolayers of four different PS-PEG materials at 25 °C. Milli-Q-purified water (18 MΩ·cm resistivity) was used as the subphase. The monolayer compression rate was 3 mm/min.

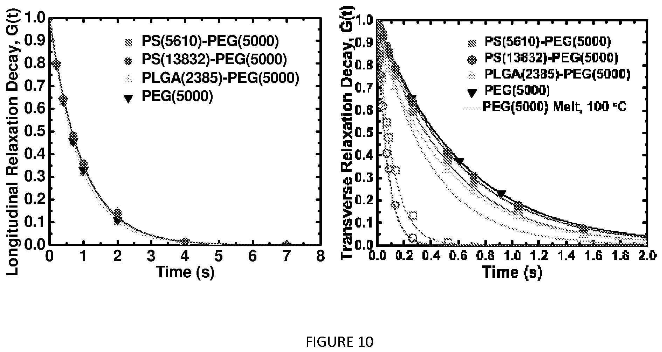

Figure 10. (a) Longitudinal relaxation decay curves for PEG protons at 25 °C. Solid curves are fits to a mono-exponential decay function ($G(t) = \exp(-t/T_1)$). (b) Transverse relaxation decay curves for PEG protons at 25 °C. Spectra from PS(5610)-PEG(5000) and PS(13832)-PEG(5000) micelles exhibited two PEG peaks (a sharp peak at ~ 3.61 ppm, and a broad peak at ~ 3.56 ppm). The decay curves of these peaks were separately fit with a mono-exponential decay function. Open symbols represent broad PEG peaks, and filled symbols represent sharp PEG peaks. Spectra from PEG(5000) and PLGA(2385)-PEG(5000) exhibited single PEG peaks. The decay curve of PEG(5000) was fit with the mono-exponential function, and that of PLGA(2385)-PEG(5000) was fit with a bi-exponential function ($G(t) = a \cdot \exp(-t/T_{21}) + (1-a) \cdot \exp(-t/T_{22})$).

**Table 3.** Best fit $T_1$ and $T_2$ values from Figure 10. [†] Fractions of PEG segments contributing to the sharp and broad PEG peaks out of the total number of PEG segments available in the system, estimated based on pyridine internal reference. [‡]The coefficient of the first term of the bi-exponential decay function, a. Also shown for comparison is a predicted $T_2$ value for PEG(5000) melt at 100 °C.

| Polymer | PEG-Sharp $T_1$ (s) | PEG-Broad $T_1$ (s) | PEG-Sharp $T_2$ (s) | PEG-Broad $T_2$ (s) | PEG-Sharp Fraction[†] | PEG-Broad Fraction[†] |
|---|---|---|---|---|---|---|
| PS(5610)-PEG(5000) | 0.9329 | 0.9264 | 0.5211 | 0.1139 1 | $11.6 \pm 1.6\%$ | $80.6 \pm 4.5\%$ |
| PS(13832)-PEG(5000) | 0.9133 | 0.9280 | 0.5900 | 0.07216 | $34.1 \pm 1.6\%$ | $62.6 \pm 2.3\%$ |

| Polymer | PEG $T_1$ (s) | PEG $T_{21}$ (s) | PEG $T_{22}$ (s) | a[≠] | PEG Fraction[†] |
|---|---|---|---|---|---|
| PLGA(2385)-PEG(5000) | 0.8800 | 0.5225 | 0.02114 | 0.9071 | $90.3 \pm 2.4\%$ |

| Polymer | PEG $T_1$ (s) | PEG $T_2$ (s) | PEG Fraction[†] |
|---|---|---|---|
| PEG(5000) | 0.8960 | 0.6603 | $104.8 \pm 7.2\%$ |
| PEG(5000) Melt, 100 °C | N/A | 0.3838 | N/A |

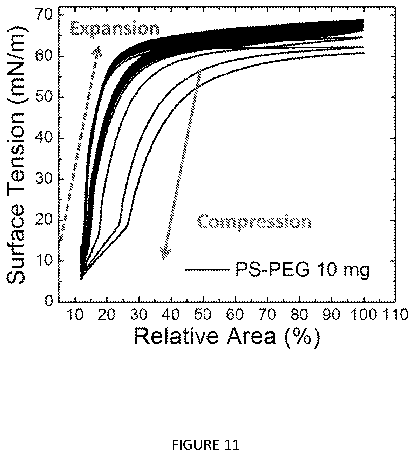

**Figure 11.** Surface tension-area isotherms for a water-spread PS(5610)-PEG(5000) monolayer during repeated compression-expansion cycles. The subphase solution contained 150 mM NaCl, 2mM $CaCl_2$, and 0.2 mM $NaHCO_3$ at pH 7.0 - 7.4 and 25 °C. The monolayer was compressed/expanded at a rate of 50 mm/min. At this rate, it took 7.18 minutes to perform one compression-expansion cycle. In this measurement, total 50 continuous compression-expansion cycles were applied.

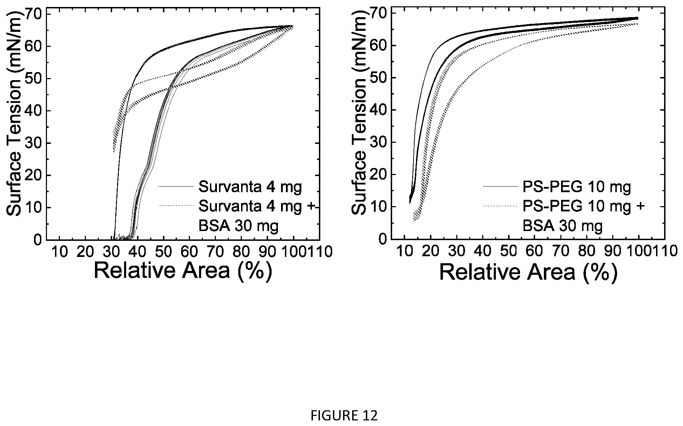

**Figure 12.** Surface tension-area isotherms for (a) Survanta (4 mg) with and without added BSA (30 mg), and (b) water-spread PS(5610)-PEG(5000) micelles (10 mg) with and without added BSA (30 mg) during repeated compression-expansion cycles. The data displayed represent the last 10 compression-expansion cycles of a total 50 continuous cycles performed after spreading Survanta or PS-PEG micelles. BSA (30 mg) was spread separately onto a pre-spread Survanta (4 mg) or PS-PEG micelle (10 mg) monolayer. The subphase solution used contained 150 mM NaCl, 2 mM $CaCl_2$ and 0.2 mM $NaHCO_3$ (pH 7.0 - 7.4, 25 °C). The monolayer was compressed/expanded at a rate of 50 mm/min; one compression-expansion cycle took 7.18 minutes.

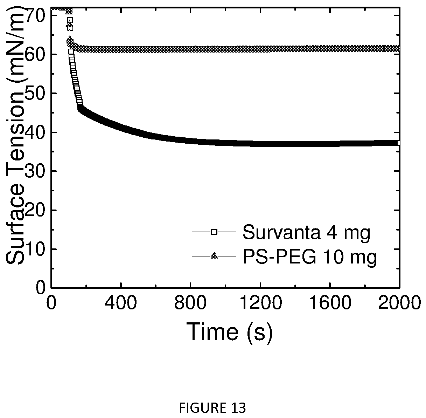

**Figure 13.** Rates of the surface tension lowering effects of PS(5610)-PEG(5000) micelles vs. Survanta measured after the spreading of the respective compounds at 100 s. The subphase solution used contained 150 mM NaCl, 2 mM $CaCl_2$ and 0.2 mM $NaHCO_3$ (pH 7.0 - 7.4, 25 °C).

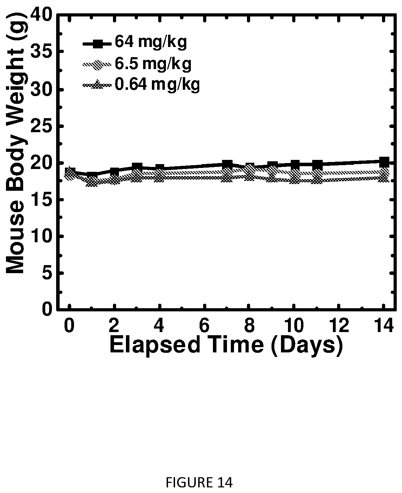

**Figure 14.** Mouse body weight measured as a function of time following intratracheal injection of different doses of PS(4418)-PEG(5000) micelle polymer lung surfactants. Each dose group consists of one mouse.

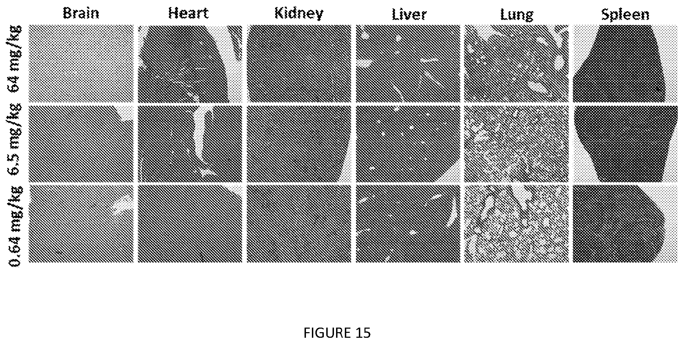

**Figure 15.** H&E-stained histological sections (1600 $\mu$m $\times$ 1200 $\mu$m) of mouse organs taken at 2 weeks after intratracheal injection of 1.6 mg PS(4418)-PEG(5000) micelle polymer lung surfactants.

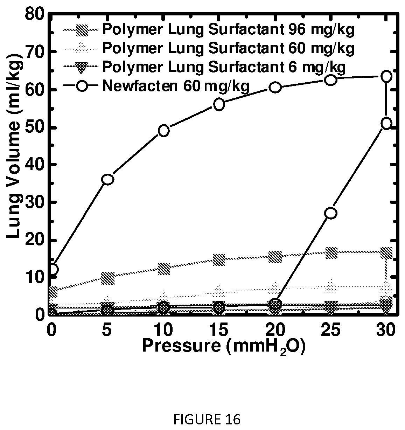

**Figure 16.** *Ex vivo* P-V lung mechanics of 27-day gestation rabbit fetus lungs following injection of various doses of PS(4418)-PEG(5000) lung surfactants. Each group consisted of five rabbit fetuses.

## DESCRIPTION

The present invention is defined in the appended set of claims. For the purposes of promoting an understanding of the principles of the present disclosure, reference will now be made to the embodiments illustrated in the drawings, and specific language will be used to describe the same. It will nevertheless be understood that no limitation of the scope of this disclosure is thereby intended.

In response to the unmet need, we developed polymer lung surfactants as a possible solution to these problems. Our newly-developed fully-synthetic polymer-based lung surfactants can be produced at significantly lower costs, and enable to use far simpler non-invasive non-physician-assisted aerosol delivery procedures.

## Surface Tension & Lung Surfactant

Developing new SRT treatment requires understanding how the lung functions are impaired if the lungs are underdeveloped. The lungs of preterm/premature infants differ from those of full-grown healthy infants by not having completely developed alveolar structures with low or no production of lung surfactants. The underdeveloped alveolar structure, although it negatively affects the breathing of the infant, is not the major cause of RDS. In fact, even at full gestation, mice and other mammals are born without fully developed lung alveolar structures, and do not suffer from RDS.[1] This suggests that the main reason for RDS is not the structural deficiency but the deficiency of a functional compound, lung surfactants. The lung surfactants, composed of 80% lipids and 20% proteins, are surface active agents that play a crucial role in the biophysical functioning of the lungs by reducing the surface tension of the alveolar lining fluid (ALF; also referred to as epithelial lining fluid (ELF)).[2] When lung surfactants are deficient in the lungs, the reduction of the ALF surface tension is hindered, and this situation creates a high pressure difference between the lung's air sacs (alveoli).

During breathing cycles, the radius (R) of an alveolus changes, and the pressure ($\Delta P$) within the alveolus also changes accordingly following the Young-Laplace relation:

$$\frac{\gamma}{R} = \Delta P.$$

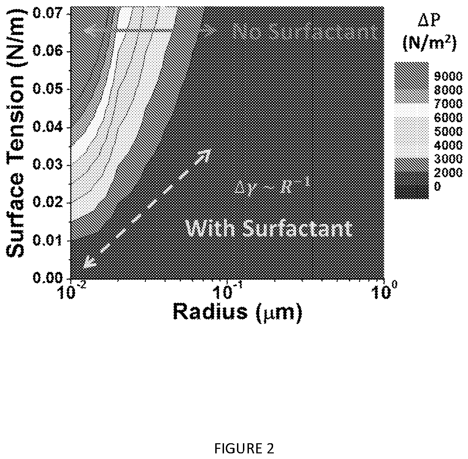

Figure 2 shows calculations of how $\Delta P$ varies as a function of $\gamma$ and R. When no surfactant is present in the lungs, the ALF surface tension is constant regardless of R ($\approx 0.069$ N/m at 37 °C), and thus the pressure can become extremely high ($\approx 9,000$ N/m$^2$) when the alveolus is contracted to a minimum size (0.02 - 0.03 mm diameter), as shown with the solid red line in Figure 2. Under this high pressure, a larger alveolus will continue to become larger, and a smaller alveolus will constantly become smaller, eventually resulting in a complete collapse of the alveolar structures. When lung surfactants are present, the surface tension is continuously reduced (even < 0.01 N/m) as the radius of the alveolus is decreased (see the dotted green line in Figure 2). This mechanism prevents pressure variation (and thus lung collapse) during breathing cycles.

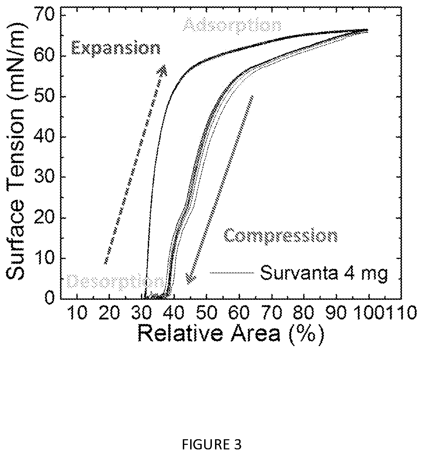

The surface tension decrease of the ALF with decreasing radius of the alveolus is caused by the repulsion among the lipid molecules in the lung surfactant system. Because of their amphiphilic nature, lipids have a strong tendency to adsorb to the air/ALF interface. During the exhalation, the decrease in alveolar radius and thus its surface area causes the surface-adsorbed lipid molecules to become laterally compressed. The pressure produced by these compressed lipids causes a decrease in the surface tension as demonstrate in Figure 3. When the surface tension is decreased to close to zero, the surface-adsorbed lipids becomes unstable, and starts to desorb into the ALF subphase; This lipid desorption produces oscillations in the surface tension profile near 0 mN/m as demonstrated in Figure 3.[12] During the inhalation, the surface area of an alveolus increases, producing uncoated surfaces. With the aid of surfactant proteins B and C (SP-B and SP-C, respectively), desorbed lipids in the subphase re-adsorb to the ALF surface during the monolayer expansion.[13] The continuous desorption and re-adsorption of lipids during repeated compression-expansion cycles are only possible in the presence of SP-B and SP-C proteins, and are the key processes that any SRT therapeutics should be able to replicate. Earlier clinical trials of SRT with lipid-only formulations were largely unsuccessful because in the absence of SP-B/SP-C the desorbed lipids produced during compression do not re-adsorb to the surface of the ALF.[14,15] The first synthetic lung surfactant, Exosurf (GlaxoSmithKline), containing 85 wt.% dipalmitoylphosphatidylcholine (DPPC), 9 wt.% hexadecanol and 6 wt.% tyloxapol, is discontinued because of its poor efficacy in treating RDS; the basic reason for this poor performance was that this formulation did not contain any protein components.

The current trend in SRT research is to develop synthetic replacements to natural surfactant proteins. To date, there is only one lung surfactant product in this category that is under clinical evaluation, Surfaxin (formerly Discovery Labs; now Windtree Therapeutics); this formulation contains a synthetic polypeptide (KL$_4$) designed to mimic the functions of natural surfactant proteins.[16,17] A disadvantage of Surfaxin, however, was its lower stability against temperature variation, compared to animal-extracted lung surfactants (such as Survanta, Curosurf, Infasurf, etc.). Surfaxin cannot be returned to refrigeration once submitted to warming procedures (e.g., 15 minutes at 44 °C in a dry block heater followed by cooling to body temperature) prior to use.[18] Clinical trials of Surfaxin showed marginal improvement in reducing morbidity and mortality compared to bovine-extracted Survanta (AbbVie) or porcine-extracted Curosurf (Chiesi Pharma).[19,20] However, it should be noted that the "Surfaxin in Therapy Against RDS (STAR)" trial was terminated early due to low enrollment (only half of the projected data was acquired), and the "Safety and Assessment of Effectiveness of Lucinactant versus Exosurf in a Clinical Trial (SELECT)" trial has been criticized for its biased assessment.[21,22] Therefore, it cannot be stated that Surfaxin produces better therapeutic outcomes over animal-extracted RDS therapeutics, or vice versa. To date, there is no single lung surfactant product that shows clear cut superior results relative to others.[23] In April 2015, Discovery Labs (renamed in April 2016 to Windtree Therapeutics) ceased production of Surfaxin to restructure the company's focus to an aerosolized formulation, Aerosurf. So currently all marketed SRT therapeutics are animal-derived products.

Cost comparison among different SRT therapeutics are not straightforward. Several factors determine the overall treatment cost including the number of intratracheal injections, the duration of mechanical ventilation, the preparation needed, the dosage administered, etc. [18] The cost of the lung surfactant drug itself is only 2 - 3% of the total SRT treatment cost in the United States. The majority of the treatment cost is related to the complex procedures associated with lung surfactant administration.[24,25] Therefore, the two main factors that prevent world-wide use of the SRT technology, i.e., high cost, and complex delivery procedures, are interconnected. Solving this decades-old problem perhaps requires "thinking out of the box". For this reason, our laboratory has been studying the behaviors of synthetic polymer surfactants (particularly, PEG-based amphiphilic block copolymers) at the air-water interface for the past few years. Polymer lung surfactants are attractive as SRT therapeutics because they can be mass produced at low costs, can easily be aerosolized (in both liquid and dry powder forms), and are chemically stable (polymer formulation would have long shelf lives). In the course of this study, we have developed design criteria for polymer lung surfactants; a successful lung surfactant candidate should (1) be biocompatible/biodegradable, (2) produce an extremely low surface tension at high compression (< 10 mN/m) repeatedly during multiple compression-expansion cycles, (3) be protein resistant, and (4) spread fast on water (within < 2 minutes).

**Polymer Lung Surfactant Criterion #1: Biocompatibility/Biodegradability**

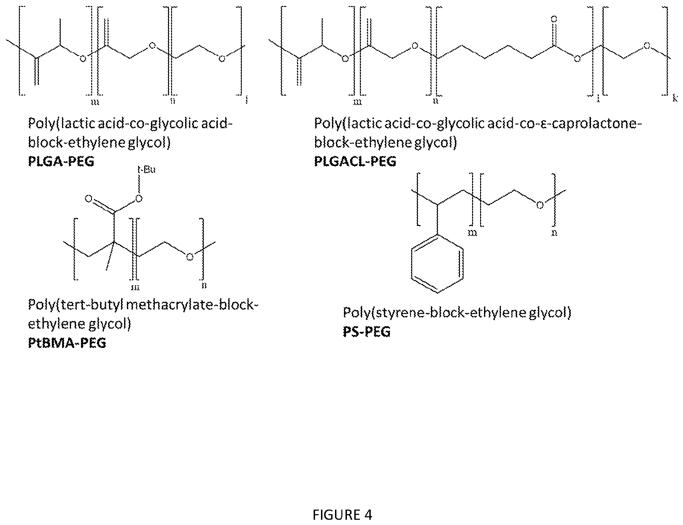

The chemical structures of candidate polymer lung surfactant materials tested are shown in Figure 4. Their molecular characteristics are also summarized in Table 1. The first two materials in the list are a well-known FDA-approved biodegradable block copolymer, poly(lactic acid-*co*-glycolic acid-*block*-ethylene glycol) (PLGA-PEG), and its derivative, poly(lactic acid-*co*-glycolic acid-*co*-caprolactone-block-ethylene glycol) (PLGACL-PEG). The other candidate block copolymers studied are poly(tert-butyl methacrylate-*block*-ethylene glycol) (PtBMA-PEG) and poly(styrene-*block*-ethylene glycol) (PS-PEG). The PtBMA-PEG and PS-PEG materials are biocompatible, particularly, when they are formulated into micellar solutions; in micelle solutions, the PtBMA and PS blocks form hydrophobic core domains, and the PEG chains form hydrophilic corona layers. PS-PEG micelles have been studied in great detail as potential drug delivery systems. Pharmacological studies, including toxicity, biodistribution and pharmacokinetic tests suggest that PS-PEG micelles are very safe to use for biomedical applications.[26-30] Very little pharmacological data is currently available for PtBMA-PEG micelles. However, considering the similarity between PS-PEG vs. PtBMA-PEG micelles, PtBMA-PEG micelles are expected to be also highly biocompatible; data available for similar systems, such as poly(methyl methacrylate-*co*-methacryloxysuccinimide-*graft*-poly(ethylene glycol)) ("PMMA-co-PMASI-g-PEG") micelles, also support this view.[31]

**Table 1.** Molecular characteristics of polymer lung surfactant candidate materials investigated. [1] lactide:glycolide = 70:55 by mole. [2] lactide:glycolide:caprolactone = 30:29:41 by mole.

| Polymer | $M_{n,PEG}$ (g/mol) | $M_{n,PLGA/PLGACL/PS/PtBMA}$ (g/mol) | PDI |
|---|---|---|---|
| PLGA-PEG | 5,000 | 8,333[1] | 1.46 |
| PLGACL-PEG | 5,000 | 9,857[2] | 1.33 |
| PtBMA-PEG | 5,000 | 1,785 | 1.12 |
| PS(1560)-PEG(5000) | 5,000 | 1,560 | 1.10 |
| PS(2993)-PEG(5000) | 5,000 | 2,993 | 1.13 |
| PS(5610)-PEG(5000) | 5,000 | 5,610 | 1.16 |
| PS(13832)-PEG(5000) | 5,000 | 13,832 | 1.38 |

In our study, these block copolymer lung surfactants were formulated into aqueous micelle solutions. Spherical micelle structures were derived using the solvent exchange procedure.[32,33] The polymer micelle formulations were highly stable even at room temperature, and did not require any pretreatment processes before use. This is already a great advantage over lipid-based conventional lung surfactant formulations, which typically require specific storage and pretreatment procedures prior to use to obtain reproducible results. This advantage in handling characteristics alone can already contribute to effectively reducing the total treatment cost.

### Polymer Lung Surfactant Criterion #2: Extremely Low Surface Tension (or High Surface Pressure) at High Compression

Initially, we focused our study on an FDA-approved biodegradable block copolymer, PLGA-PEG. PLGA-PEG forms a well-spread film at the air-water interface, commonly referred to as a Langmuir monolayer. A Langmuir trough device was used to create an *in vitro* lung-mimicking test environment. When a sufficient amount of PLGA-PEG is spread on the water surface beyond the full coverage point, the PLGA-PEG polymers form a brush-coated insoluble film, in which the PLGA segments are anchored to the surface of water (forming a slightly glassy, insoluble polymer film), and the PEG segments are submerged to the water subphase (forming a brush layer).c In the highly laterally compressed state, PLGA-PEG reduces the surface tension of water down to close to zero because of the combined effects of PLGA glass transition and PEG brush repulsion.[35,36] The morphological and surface mechanical properties of Langmuir PLGA-PEG monolayers under various monolayer compression conditions are discussed in detail in References [34,35].

Figure 5 (a) displays the surface tension-area isotherms obtained from the Langmuir monolayers formed by PLGA-PEG and its non-glassy analogue, PLGACL-PEG; the monolayers were prepared using chloroform as the spreading solvent. At high compression, the PLGA-PEG monolayer showed a surface tension as low as about 8 mN/m. The PLGACL-PEG monolayer showed a much higher surface tension, i.e., about 40 mN/m, at maximum compression, because PLGACL is a non-glassy polymer. In real therapeutic applications, chloroform cannot be used as the spreading solvent. The formulation has to be water-based. In aqueous solutions, PLGA-PEG and PLGACL-PEG exist in the form of micelles. The surface tension-area profiles of PLGA-PEG and PLGACL-PEG micelle monolayers (prepared using water as the spreading solvent) are presented in Figure 5 (b). In these water-spread situations, the surface tension behaviors of the PLGA-PEG and PLGACL-PEG monolayers were quite comparable to each other; in both cases, the lowest surface tension observed was about 50 - 60 mN/m at the highest compression level tested.

The reason why the chloroform-spread versus water-spread PLGA-PEG monolayers exhibit drastically different surface tension isotherms is that in the water-spread monolayer system the PLGA-PEG polymers remain in the micelle state, whereas in the chloroform-spread situation the polymers form a molecularly-spread ("anchor-brush") monolayer. The water-spreading method has been tested with many different PLGA-PEG samples having widely varying overall molecular weights (3.5 - 28.6 kg/mol) and block compositions (28.4 - 74.3 PEG wt.%), however, none of the samples exhibited a sufficiently low surface tension (typically always > 45 mN/m) under high compression; water-spread PLGA-PEG micelles always remained intact following initial spreading at the air-water interface (unpublished results/manuscript in preparation).

To achieve lower surface tension with water-spread polymer lung surfactants than what was obtainable with PLGA-PEG and PLGACL-PEG, other hydrophobic chemistries have been tested. It was hypothesized that if water-spread micelles fuse and form an anchor-brush-type monolayer, the resulting monolayer would produce much lower surface tensions. To test this hypothesis, PtBMA-PEG was used. PtBMA has a tendency to wet the water surface.[37,38] The wetting (i.e., spreading) tendency of a polymer can be quantified in terms of the spreading coefficient (S):

$$S = \gamma_{\mathrm{air-water}} - \gamma_{\mathrm{air-polymer}} - \gamma_{\mathrm{polymer-water}}.$$

The more positive the value of S is, the stronger the wetting tendency is. Using the known interfacial tension values, i.e., $\gamma_{\mathrm{air-water}}$ = 72 mN/m (25 °C), $\gamma_{\mathrm{air-PtBMA}}$ = 30 mN/m, and $\gamma_{\mathrm{PtBMA-water}}$ = 18 mN/m, the spreading coefficient of PtBMA is calculated to be S = 24 mN/m > 0. The spreading coefficient of PLGA is about 10 mN/m. These numbers indicate that PtBMA-PEG has a stronger tendency to spread on water than PLGA-PEG. Therefore, there is a higher chance that a PtBMA-PEG micelle monolayer undergoes spontaneous transformation into a laterally uniform, anchor-brush-type monolayer.

The surface tension properties of chloroform-spread vs. water-spread PtBMA-PEG monolayers were investigated; the results are presented in Figure 6 (a). Interestingly, in the PtBMA-PEG case, even the water-spread monolayer exhibited very low surface tension (< 10 mN/m at high compression), similarly to the chloroform-spread monolayer. Both the chloroform-spread and water-spread isotherms showed a transition to a (pseudo-)plateau around a surface tension of about 48 mN/m, which can be attributed to the PtBMA anchor block as it forms a continuous film layer; this interpretation is supported by the fact that the surface tension at the plateau transition is identical to the value estimated from the spreading coefficient for PtBMA, i.e., ($\gamma_{\mathrm{polymer-air}}$ + $\gamma_{\mathrm{polymer-water}}$) = $\gamma_{\mathrm{air-water}}$ - S = 72 - 24 = 48 mN/m. This presents unmistakable evidence that the core domains of PtBMA-PEG micelles melt and merge into a film when the micellar monolayer is laterally compressed. This interpretation is also supported by the observation that the plateau transition became more pronounced as the temperature was increased (Figure 6 (b)); at higher temperatures, the micelle fusion process is facilitated because of the increased mobility of the PtBMA segments.

PS-PEG represents the opposite to PtBMA-PEG in terms of its water wetting characteristics. PS has a negative spreading coefficient, S = -8 mN/m;[39,40] it has a tendency to dewet from the water surface. This dewetting tendency of PS prevents the PS-PEG copolymers from forming a laterally uniform anchor-brush monolayer, even when spread from chloroform. This distinguishes PS-PEG from other polymers studied (PLGA-PEG, PLGACL-PEG, and PtBMA-PEG). In the literature, it has been documented that PS-PEG forms surface micelles on water following spreading using an organic solvent, typically, chloroform.[41-47] Also, chloroform-spread PS-PEG polymers have been shown to exhibit low surface tension (< 10 mN/m) at high compression. Prior to our investigation, it was unknown whether water-spread PS-PEG micelle monolayers would be able to produce similar low surface tension. The answer is shown in Figure 7.

Because of the dewetting of PS at the air-water interface, both chloroform-spread and water-spread PS-PEG monolayers contain unfused micelles. However, the surface micelles formed by spreading a PS-PEG solution in chloroform onto the water surface are expected to be anisotropic in molecular morphology because of the asymmetry of the air-water interface, whereas in bulk water solution, an isotropic spherical micelle morphology is typically obtained (Figure 8 (c)). This morphological difference seems to produce a large difference in the shape of the surface tension-area isotherm (Figure 7). Most importantly, the water-spread PS-PEG micelle monolayer was found to satisfy the low surface tension requirement for potential use in SRT; this formulation was able to produce an extremely low surface tension ($\approx 0$ mN/m) at high compression (Figure 7).

We examined how changing the PS block size influences the morphological and surface tension properties of the block copolymer. Three additional PS-PEG samples were prepared having different PS block molecular weights at a fixed PEG molecular weight of 5,000 g/mol: PS(1560)-PEG(5000), PS(2993)-PEG(5000), and PS(13832)-PEG(5000). As shown in Figure 8 (and also summarized in Table 2), the PS block molecular weight significantly influenced the micelle size.

**Table 2.** Diameters of PS-PEG micelles as determined by TEM (Figure 8) or DLS.

| Block Copolymer | Micelle Diameter Determined by TEM | Micelle Diameter Determined by DLS |
| --- | --- | --- |
| PS(1560)-PEG(5000) | 14.5 $\pm$ 1.7 nm (excluding elongated micelles) | 34.5 $\pm$ 2.0 nm |
| PS(2993)-PEG(5000) | 16.00 $\pm$ 4.7 nm | 23.2 $\pm$ 2.0 nm |
| PS(5610)-PEG(5000) | 20.0 $\pm$ 1.7 nm | 27.3 $\pm$ 0.2 nm |
| PS(13832)-PEG(5000) | 45.3 $\pm$ 2.7 nm | 55.8 $\pm$ 0.3 nm |

The surface tension-area isotherms were measured for the four different PS-PEG materials (both chloroform-spread and water-spread). The data are presented in Figure 9. In this figure, for clarity, the isotherm data are presented in the form of log surface pressure versus log surface area per chain plots. Here, the surface pressure ($\pi$) is defined as

$$\pi = \gamma_{air-water} - \gamma_{air-polymer-water} = \gamma_{air-water} - (\gamma_{air-polymer} + \gamma_{polymer-water})$$

where $\gamma_{air-polymer-water}$ is the surface tension of the polymer-coated air-water interface. For constructing a full surface pressure-area isotherm curve for each monolayer type over a large range of monolayer area, it was necessary to perform multiple (2 to 3) measurements in different ranges of monolayer areas because of the size limitation of the Langmuir trough. Interestingly, the isotherms obtained from chloroform-spread monolayers over different areas superimposed closely on one another without breaking (Figure 9). On the other hand, as also shown in the figure the curves from different areas for water-spread monolayers were disjointed, which suggests that the water-spreading procedure caused some loss of material (PS-PEG micelles) to the subphase; when the polymers were spread from chloroform solutions, the loss of material to the subphase was negligible.

Chloroform-spread PS-PEG monolayers exhibited similar isotherm profiles at surface pressures < about 10 mN/m, regardless of the PS block molecular weight (Figure 9 (a)). When compressed beyond the 10 mN/m surface pressure level, higher PS molecular weights produced steeper rises in surface pressure for the chloroform-spread monolayers. We suspect that this observation is due to the fact that higher molecular weight PS segments result in larger-sized core domains for the PS-PEG surface micelles. The surface mechanical properties of chloroform-spread PS-PEG surface micelles have previously been studied by other researchers.[43,44] Unlike the chloroform-spread cases, the surface pressures of water-spread PS-PEG monolayers did not exhibit a monotonic trend with respect to the PS block molecular weight. One notable observation was that in water-spread systems, maximum surface pressure was achieved at an intermediate PS block molecular weight; the steepest rise of surface pressure during compression was observed with the water-spread PS(5610)-PEG(5000) micelle monolayer (Figure 9 (b)). Interestingly, the water-spread PS(13832)-PEG(5000) monolayer exhibited the lowest maximum surface pressure among all systems tested (Figure 9 (b)). The maximum surface pressure of water-spread PS(13832)-PEG(5000) (10 - 20 mN/m) was comparable to those of water-spread PLGA-PEG and PLGACL-PEG.

It is interesting to note that although three water-spread monolayer systems examined (PS-PEG, PLGA-PEG and PLGACL-PEG micelle monolayers) are similar in morphology (i.e., these monolayers are all composed of non-mergeable micelles), only PS-PEG micelles are able to produce a high surface pressure (> 40 mN/m) under high compression. These results indicate that PS-PEG micelles are significantly more hydrophobic (than PLGA-PEG or PLGACL-PEG micelles) and are thus more strongly pinned to the air-water interface, which makes PS-PEG micelles more stable against submerging and thus the PS-PEG micelle monolayer more mechanically resistant to lateral compression. This explanation might seem unlikely if one only considers that, regardless of the hydrophobic material the block copolymer is made of (PS, PLGA, or PLGACL), the micelle corona is composed of the same polymer (PEG); that is, how can PS-PEG micelles be more hydrophobic than PLGA-PEG/PLGACL-PEG micelles when these different micelle types share a common PEG corona structure? We suspect that the difference occurs due to the fact that PS is, in fact, more strongly hydrophobic than PLGA (or PLGACL) ($\gamma_{PS-water} \approx 41$ mN/m, and $\gamma_{PLGA-water} = 25$ mN/m)[34]; as a result, (unlike PLGA-PEG and PLGACL-PEG micelles in which the PEG chains form fully hydrated brush layers) the PEG chains in PS-PEG micelles exist in a collapsed state in order to minimize the unfavorable exposure of the PS material to water molecules, which ultimately renders PS-PEG micelles overall more hydrophobic than PLGA-PEG or PLGACL-PEG micelles. Therefore, micelle hydrophobicity is an important criterion for designing/selecting an appropriate material for use as a lung surfactant.

Further study was performed to confirm the collapsed conformation of the PEG chains in PS-PEG micelles. Specifically, for this purpose, the mobilities of micellar PEG brush segments were measured for PS-PEG and PLGA-PEG micelles using the *in situ* NMR spin relaxation technique; collapsed PEG corona chains of PS-PEG micelles are expected to exhibit a significantly reduced mobility than fully hydrated PEG corona chains of PLGA-PEG micelles. From NMR spin relaxation measurements, it is possible to determine two real-time constants, i.e., the longitudinal relaxation time ($T_1$), and the transverse relaxation time ($T_2$). $T_1$ is related to the chemical structure ("fast mode"), and $T_2$ is related to the configuration ("slow mode") of the chain segment.[48] Between PS-PEG and PLA-PEG micelles, it is expected that the PEG $T_1$ values are identical, whereas their $T_2$ values are significantly discrepant. NMR measurements were performed on four representative systems: PS(5610)-PEG(5000), PS(13832)-PEG(5000) and PLGA(2385)-PEG(5000) micelles, and a PEG(5000) homopolymer in heavy water. For PS-PEG micelles, two separate PEG proton peaks were observed (a sharp ("hydrated PEG") peak at ~ 3.61 ppm, and a broad ("collapsed PEG") peak at ~ 3.56 ppm). These two peaks were separately analyzed for $T_1$ and $T_2$. The results are displayed in Figure 10.

As shown in Figure 10, all four samples tested exhibited an identical PEG $T_1$ value, which confirms the validity of the measurements. To the contrary, the measured PEG $T_2$ values varied significantly from sample to sample. To provide a scale of the PEG mobility, the $T_2$ value for 5 kg/mol PEG homopolymer melt at 100 °C was calculated; at this condition, PEG has a rouse time of 281.52 ps, which translates to $T_2 = 0.3838$ s.[49] Hydrated PEG chains are expected to have longer $T_2$ values than 0.3838 s, because of their higher mobilities. The $T_2$ value for hydrated free PEG(5000) chains was estimated to be 0.6604 from a fitting of the transverse decay curve to a mono-exponential function, $G(t) = \exp(-t/T_2)$. The transverse decay curve of PLGA(2385)-PEG(5000) micelles was fit better with a bi-exponential function, $G(t) = a \cdot \exp(-t/T_{21}) + (1-a) \cdot \exp(-t/T_{22})$, because the mobilities of PEG segments vary depending on the proximity of the PEG segment to the grafting surface. $T_{21}$ corresponded to PEG segments distant from the grafting surface, which were largely responsible for the overall signal intensity (a = 0.9071). $T_{22}$ corresponded to PEG segments close to the grafting surface. The $T_{21}$ value of PLGA-PEG micelles was higher than that of PEG melt and slightly lower than that of hydrated PEG(5000), which indicates that the PEG corona chains of PLGA-PEG micelles were indeed fully hydrated. In the PS-PEG micelle cases, NMR spectra exhibited two separate PEG peaks. These two PEG peaks were separately fit with a mono-exponential function. The $T_2$ values obtained from the decay curves of the sharp PEG peaks of PS-PEG micelles were comparable to the $T_{21}$ value obtained from PLGA-PEG micelles, which suggests that the sharp PEG peaks corresponded to hydrated PEG segments of PS-PEG micelles. However, the $T_2$ values obtained from the broad PEG peaks of PS-PEG micelles were very small, even smaller than the $T_2$ value obtained from PEG melt, which unambiguously indicates that, in PS-PEG micelles, substantial portions of PEG segments existed in a collapsed state (because of the strong hydrophobicity of the PS material). This result is also consistent with previous reports of collapsed PEG brush structures in poly(butadiene-*block*-ethylene glycol) PB-PEG) micelles ($\gamma_{PB-water} = 45.9$ mN/m) based on cryo-TEM and small angle neutron scattering (SANS) experimentation and self-consistent field (SCF) theoretical consideration.[50,51]

**Table 3.** Best fit $T_1$ and $T_2$ values from Figure 10. [†] Fractions of PEG segments contributing to the sharp and broad PEG peaks out of the total number of PEG segments available in the system, estimated based on pyridine internal reference. [‡]The coefficient of the first term of the bi-exponential decay function, a. Also shown for comparison is a predicted $T_2$ value for PEG(5000) melt at 100 °C.

| Polymer | PEG-Sharp $T_1$ (s) | PEG-Broad $T_1$ (s) | PEG-Sharp $T_2$ (s) | PEG-Broad $T_2$ (s) | PEG-Sharp Fraction[†] | PEG-Broad Fraction[†] |
|---|---|---|---|---|---|---|
| PS(5610)-PEG(5000) | 0.9329 | 0.9264 | 0.5211 | 0.1139 | 11.6 ± 1.6% | 80.6 ± 4.5% |
| PS(13832)-PEG(5000) | 0.9133 | 0.9280 | 0.5900 | 0.07216 | 34.1 ± 1.6% | 62.6 ± 2.3% |

| Polymer | PEG $T_1$ (s) | PEG $T_{21}$ (s) | PEG $T_{22}$ (s) | a[≠] | PEG Fraction[†] |
|---|---|---|---|---|---|
| PLGA(2385)-PEG(5000) | 0.8800 | 0.5225 | 0.02114 | 0.9071 | 90.3 ± 2.4% |

| Polymer | PEG $T_1$ (s) | PEG $T_2$ (s) | PEG Fraction[†] |
|---|---|---|---|
| PEG(5000) | 0.8960 | 0.6603 | 104.8 ± 7.2% |
| PEG(5000) Melt, 100 °C | N/A | 0.3838 | N/A |

Further, it is very interesting to note that PS(13832)-PEG(5000) micelles have a higher fraction of hydrated PEG segments compared to PS(5610)-PEG(5000) micelles. The absolute concentrations of hydrated vs. collapsed PEG segments of PS-PEG micelles could be determined using an NMR signal from pyridine added as an internal standard. PS(13832)-PEG(5000) micelles were found to have a significantly higher proportion of hydrated PEG segment (34.1 ± 1.6 %) than PS(5610)-PEG(5000) micelles (11.6 ± 1.6 %) (Table 3). These results clearly support that (for some reason that is yet unclear) PS(13832)-PEG(5000) micelles are less hydrophobic than PS(5610)-PEG(5000) micelles and therefore expected to be less strongly bound to the air-water interface. Indeed, as shown in Figure 9, water-spread PS(13832)-PEG(5000) micelle monolayers were found to be able to produce only relatively low surface pressures even under high compression ($\approx$ 10 mN/m).

Overall, our investigation has now led to an identification of a very promising class of candidate materials that have the desired surface tension/pressure properties for potential lung surfactant applications: the PS(5610)-PEG(5000) block copolymer formulated in the form of aqueous micelles. Aqueous micelle solutions of PS(5610)-PEG(5000) exhibit excellent colloidal stability over a long period of time; a PS(5610)-PEG(5000) micelle sample was confirmed to reproduce the same surface pressure-area profile after being stored at room temperature for over 3 months (data not shown).

Water-spread PS-PEG and PtBMA-PEG monolayers are equally promising in terms of their surface tension properties. However, no pharmacological data is currently available to gauge the in vivo safety of the PtBMA-PEG micelle material. For this reason, the PS-PEG micelle system is more favored for SRT applications. Dynamic hysteretic characteristics of the water-spread PS(5610)-PEG(5000) monolayer system were further characterized during continuous compression-expansion cycles. The results are shown in Figure 10. The PS-PEG monolayer exhibited a pronounced surface tension hysteresis during compression-expansion cycles and a very low minimum surface tension ($\approx$ 0 mN/m) at highest compression, similarly to what was observed with a commercial lung surfactant, Survanta (see Figure 3 for comparison). After the first three compression-expansion cycles, the hysteresis loops in the compression-expansion isotherms showed a closed shape (Figure 11), which suggests that there was little loss of polymer material to the subphase during the repeated compression-expansion cycles. It is notable that in the PS-PEG monolayer system this combination of hysteretic and reversible characteristics were readily achieved even without aid of added proteins, which we consider to be an advantageous aspect of using a polymeric agent for controlling the air-water interface. It appears that PS-PEG micelles do not desorb from the water surface; so, unlike natural lung surfactants, polymer lung surfactants do not require proteins for their functions.

**Polymer Lung Surfactant Criterion #3: Protein Resistance**

Respiratory failure due to deactivated lung surfactants caused by an increase level of serum albumin proteins (or other surface active agents) is called Acute Respiratory Distress Syndrome (ARDS).[52,53] Therapeutics developed for treatment of nRDS are not effective in treating ARDS, because of the deactivation of injected lung surfactants.[54,55] The protein resistance characteristics of PS-PEG micelle lung surfactants were evaluated.

First, we tested how a commercial lung surfactant, Survanta (AbbVie), responds to an addition of a surface active protein, Bovine Serum Albumin (BSA). As shown in Figure 12 (a), BSA deactivated Survanta. Upon addition of BSA, Survanta lost the capability to reduce the surface tension down close to zero; the minimum surface tension increased to about 26 mN/m. To the contrary, the surface tension lowering activity of PS-PEG micelles was largely unaffected by added BSA (Figure 12 (b)). PS-PEG micelle lung surfactants might also be useful for ARDS treatment.

**Polymer Lung Surfactant Criterion #4: Fast Spreading on Water**

The surface tension lowering effects of any SRT therapeutics should occur within a few minutes after initial application; otherwise, the patient will face a life-threatening condition. We tested whether PS-PEG micelles spontaneously spread very fast on water surface. As demonstrated in Figure 13, we confirmed that the surface tension lowering effect of PS-PEG micelles reached its steady-state level within about 100 s following the spreading of the polymers onto the water surface. This is a much shorter induction time than what was observed with Survanta (about 800 s). The spreading speed should not be exactly equal to the speed of therapeutic effect. Nevertheless, the fast spreading rate of PS-PEG micelle lung surfactants is an advantageous feature that raises the feasibility of its application in SRT.

**Safety of Intratracheally Injected PS-PEG Micelles in Adult Mice**

A preliminary study was performed to evaluate the safety of intratracheally administered PS-PEG lung surfactants in adult mice. Three polymer dose levels were tested: 0.64, 6.5 and 64 mg polymer per kg mouse body weight (20 microliters of 0.6, 6 and 60 mg/ml PS-PEG micelle solutions were respectively injected to mice). The polymer used was PS(4418)-PEG(5000); the overall molecular weight and block composition were selected based on *in vitro* results discussed earlier. Separate experiments were conducted to confirm that this PS(4418)-PEG(5000) material satisfied four polymer lung surfactant performance criteria discussed earlier. Polymer lung surfactants were injected through surgical incision in the trachea. Mice were monitored for indications of toxicity (weight loss and behavioral symptoms) for 14 days since the time of injection.

Mouse body weights are presented in Figure 14. The initial weight loss observed during the first 1 or 2 days is perhaps due to the surgical incision. Once mice recovered from the surgery, they exhibited normal behavior and started steadily gaining body weight. At all polymer doses tested, no signs of toxicity was observed in mice during the 14 day period. At day 14 post injection, mice were sacrificed, and major organs (brain, heart, kidney, liver, lung, and spleen) were collected for histological analysis. A blinded histopathological evaluation of H&E-stained organ specimens was performed by a histopathology expert, who confirmed that all organs were normal; representative images of the H&E-stained tissue sections are presented in Figure 15.

**Efficacy of Intratracheally Injected PS-PEG Micelles in Preterm Rabbit Lungs**

Pressure(P)-volume(V) measurements using *ex vivo* lung models (excised animal lungs) are a common method of evaluating the efficacy of an RDS therapeutic. Due to the high reproducibility of lung P-V mechanics among independent tests, *ex vivo* P-V testing is an FDA-approved method for quality control(QC)/quality assurance(QA) of animal-extracted lung surfactant products.[2] The animal model used in this study was preterm 27-day gestation New Zealand White Rabbit fetuses. A commercial bovine-extracted lung surfactant product, Newfacten (Yuhan Corporation) was also tested as positive control.

The same polymer used in the *in vivo* toxicity study in mice, PS(4418)-PEG(5000), was also used in the present *ex vivo* efficacy study in rabbit fetuses. Three different polymer doses were used: (0,) 6, 60 and 96 mg polymer per kg rabbit fetal body weight, achieved by injecting 1.5 ml per kg body weight of 0.6, 6 and 60 mg/ml PS-PEG micelle solutions, respectively, to rabbit fetus lungs (the rabbit fetuses weighed between 20 and 30 g); 96 mg/kg was the maximum possible dose achievable with a polymer concentration of 64 mg/ml (which is the highest polymer concentration achievable with our current solvent exchange procedure) at the optimal liquid installation volume of 1.5 ml per kg body weight.

The P-V profiles of rabbit fetus lungs following PS-PEG (or Newfactan) injection are displayed in Figure 16. At a glance, it appears that PS-PEG lung surfactants exhibit a lower efficiency in increasing the lung compliance and thus reducing the respiratory work than the commercial formulation, Newfactan, at an identical mass dose. However, these results are promising in that they demonstrate dose-dependent effects of PS-PEG on improving lung compliance. Further, the slope of the PS-PEG dose dependence trend suggests that PS-PEG indeed has great potential for use in RDS therapy at its optimal dose. Consequently, further study is needed to identify the optimal dose of PS(4418)-PEG(5000) required for sufficient therapeutic effect.

It should also be noted that a recent study suggested that viscosity plays a critical role in determining the performance of a lung surfactant, particularly because viscosity controls the fluid distribution during the endotracheal instillation process.[56,57] Our PS(4418)-PEG(5000) formulation at a polymer concentration of 64 mg/ml has a bulk shear viscosity of 1.06 cp at 37 °C, which is not too far from the bulk shear viscosity of pure water, 0.6 cp. Also of note, a discontinued synthetic lung surfactant product, Exosurf (Glaxo-Wellcome), had a viscosity of 3 cp.[56,58,59] Computational fluid dynamics (CFD) simulations suggested that low viscosity can cause uneven distribution of lung surfactants within the lungs, whereas animal-extracted lung surfactant products (e.g., Survanta, Infasurf, Curosurf) with a shear viscosity ($\approx$ 30 cp) are typically distributed very uniformly in the lungs upon instillation.[56] Therefore, the low viscosity of the current PS(4418)-PEG(5000) formulation also suggests room for improvement.

Our data suggest that polymer lung surfactants have great potential for use in SRT. Since the initial development of animal-derived RDS therapeutics in 1980s, little further progress has been achieved in this field. Aerosol delivery and synthetic protein replacement have been the main thrust in research, but efforts have met with limited success.[60-63] Testing fully synthetic polymer materials in SRT represents a radical shift in the direction of lung surfactant research. Polymer lung surfactants may open the door to new therapeutic options for the treatment of RDS that had not previously been feasible with conventional lipid-based SRT therapeutics. Polymer lung surfactants can easily be aerosolized in liquid or powder form; in the drug delivery literature, such polymers as PS, PLGA and PLGA-PEG have been frequently used as excipients for pulmonary drug delivery.[64-69] Polymer lung surfactant formulations can also be used to co-deliver additional therapeutic agents which carry a risk of causing lung surfactant deactivation when delivered alone.

What is possibly the greatest advantage is that the reversible and hysteretic surface tension lowering effects observed with the PS-PEG and PtBMA-PEG systems could be achieved with other, wider choices of chemistries, creating opportunities for further improvements and new applications of this technology.

For the first time, the concept of using a completely synthetic polymer material as an SRT therapeutic has proposed, and its feasibility has been demonstrated. Polymer lung surfactants have the potential to address the limitations of current animal-derived lipid-based RDS therapeutics: high production/treatment costs, and complex delivery procedures. Polymer lung surfactants have far longer shelf lives, and would not require any complicated pretreatment processes prior to use in treatment. Unlike lipid-based lung surfactants, the dynamic surface active characteristics of polymer lung surfactants do not degrade even in the presence of competing surface active proteins In preliminary animal studies it was confirmed that intratracheally administered polymer lung surfactants can be tolerated (and cleared from the body) without causing damage in major organs in mice, and are capable of producing dose-dependent destiffening effects on the compliance properties of rabbit fetus lung. Further research is warranted to optimize the formulation for maximum therapeutic effect and to evaluate the detailed short- and long-term toxicology of the material.

## Experimental Procedures

### PLGA-PEG and PLGACL-PEG Synthesis

PLGA-PEG and PLGACL-PEG materials were synthesized by ring-opening polymerization using a tin catalyst. Purified poly(ethylene glycol) monomethyl ether (PEG-OH, $M_n$ = 5,000 g/mol, Sigma Aldrich) was used as the macroinitiator, and tin(II) 2-ethylhexanoate (Sigma Aldrich) was used as the catalyst. The polymerization reactions were run at 130 °C. The D,L-lactide (Lactel) and glycolide (Sigma Aldrich) monomers were twice recrystallized from toluene (Sigma Aldrich) and tetrahydrofuran (Sigma Alrdich) prior to use. The $\varepsilon$-caprolactone (Sigma Aldrich) monomer was used as received. The synthesized PLGA-PEG and PLGACL-PEG products were precipitated in 2-propanol (Sigma Aldrich) and dried under vacuum before use/storage at refrigeration temperatures.

### PtBMA-PEG and PS-PEG Synthesis

PtBMA-PEG and PS-PEG materials were synthesized by Reversible Addition-Fragmentation Chain-Transfer (RAFT) polymerization. 4-cyano-4-[(dodecylsulfanylthiocarbonyl)sulfanyl] pentanoic acid (Sigma Aldrich) was used as the RAFT agent. First, the RAFT agent was conjugated to purified poly(ethylene glycol) monomethyl ether (PEG-OH, $M_n$= 5,000 g/mol, Sigma Aldrich) by Steglich esterification.[70] The PEG-OH (1 g, 0.2 mmol), the RAFT agent (161.4 mg, 0.4 mmol), and 4-dimethylaminopyridine (Sigma Aldrich, 4.89 mg, 0.04 mmol) were mixed in 10 ml dichloromethane (Sigma Aldrich), and was kept under magnetic stirring at 0 °C. A separately prepared dicyclohexylcarbodiimide (82.5 mg, 0.4 mmol) solution in dichloromethane (5 ml) was drop-wise added to the above mixture, and was allowed to undergo reaction for 5 minutes at 0 °C and then for 3 hours at 20 °C to produce "PEG-RAFT". The as-synthesized PEG-RAFT product was first filtered through filter paper to remove the insoluble urea byproduct, and was then further purified by precipitation in hexane twice. The RAFT polymerization reaction was performed at 70 °C by mixing the PEG-RAFT, inhibitor-free styrene (Sigma-Aldrich) or tert-butyl methacrylate (Sigma-Aldrich), and a free radical initiator, azobisisobutyronitrile (Sigma-Aldrich) in dioxane (Sigma Aldrich). The resulting PtBMA-PEG and PS-PEG products were precipitated twice in hexane, and dried under vacuum. The synthesis procedure was adopted from the following reference: Benaglia, M. et al. Universal (Switchable) RAFT Agents. Journal of the American Chemical Society 131, 6914-6915, doi:10.1021/ja901955n (2009).

### Polymer Characterizations

The number averaged molecular weights ($M_n$) of the polymers were determined by $^1$H NMR spectroscopy using

a Bruker ARX NMR spectrometer (500 MHz). For $^1$H NMR measurements, polymer samples were prepared in deuterated chloroform at a polymer concentration of 5 wt.%. The polydispersity indices (PDIs) of the polymers were measured by size-exclusion chromatography (SEC) using an Agilent Technologies 12000 Series instrument equipped with a Hewlett-Packard G1362A refractive index detector and three PLgel 5 $\mu$m MIXED-C columns. Tetrahydrofuran was used as the mobile phase (kept at 35 °C, flowing at a rate of 1 ml/min). Calibration was performed using polystyrene standards (Agilent Easi Cal).

*Surface Tension-Area Isotherms*

The surface tension-area isotherms for Survanta and polymer lung surfactants were measured using a KSV 5000 Langmuir trough (51 cm x 15 cm) with double symmetric barriers. The total surface area of the trough was 780 cm$^2$, and the subphase volume was 1.3 L. Filter paper Wilhemly probe was used for surface tension measurements. Before each measurement run, the trough and the barriers were cleaned three times using ethanol and Milli-Q-purified water. The surface of water was also aspirated to remove any surface active contaminants. When the water surface was completely clean, the surface tension reading did not change during a blank compression run. Lung surfactant samples were spread onto water using a Hamilton microsyringe, i.e., by forming a microliter-sized droplet at the tip of the syringe needle and letting it contact the water surface.

*Polymer Micelle Preparation*

The solvent exchange procedure was used to prepare spherical polymer micelles. 200 mg of the polymer was first dissolved in 4 ml of acetone (Sigma Aldrich). Then 36 ml of Milli-Q-purified water (18 M$\Omega$·cm resistivity) was drop-wise added to the polymer solution at a rate of 0.05 ml/min using a syringe pump, and the mixture was kept under vigorous stirring for 24 hours. To remove the acetone, the solution was transferred to a dialysis bag (Spectra/Por 7, 50 kDa molecular weight cutoff), and dialyzed for 3 hours against 1 L Milli-Q-purified water. The reservoir was replaced with fresh Milli-Q water every hour. The micelle preparation procedure was adopted from the following reference:
Zhang, L. & Eisenberg, A. Multiple Morphologies and Characteristics of "Crew-Cut" Micelle-like Aggregates of Polystyrene-b-poly(acrylic acid) Diblock Copolymers in Aqueous Solutions. Journal of the American Chemical Society 118, 3168-3181, doi:10.1021/ja953709s (1996)

*Polymer Micelle Characterizations*

The hydrodynamic diameters of the block copolymer micelles were measured at 25 °C by Dynamic Light Scattering (DLS) using a Brookhaven ZetaPALS instrument. The scattering intensities were measured using a 659 nm laser at a scattering angle of 90 °. The hydrodynamic diameters were calculated from the measured diffusion coefficients using the Stokes-Einstein equation. For DLS measurements, the samples were diluted to guarantee single scattering, and were filtered with 0.2-$\mu$m syringe filters to remove contaminants.
Transmission Electron Microscopy (TEM) was used to image the polymer micelles. TEM specimens were prepared by placing 20 $\mu$l of a 0.01 - 0.05 mg/ml polymer micelle solution on a carbon-coated copper TEM grid (hydrophobically treated using a O$_2$ plasma cleaner). 10 $\mu$l of a 2% uranyl acetate solution was added to the sample solution already placed on the TEM grid, and the mixture was blotted using filter paper and dried. The samples thus prepared were imaged using a 200 kV FEI Tecnai 20 TEM instrument. The TEM images were analyzed using the Gatan Digital Micrograph software.

*NMR Spin Relaxation Measurements*

NMR spin relaxation measurements were performed using a Bruker Avance-III-800 Spectrometer equipped with a sample temperature control unit. PLGA-PEG and PS-PEG micelle samples were prepared using the solvent exchange procedure (described above) using D$_2$O (instead of H$_2$O) as the final solvent. The PEG homopolymer sample was prepared by directly dissolving PEG in D$_2$O. In all samples, the polymer concentration was 0.5 wt.%. The inversion recovery sequence was used for T$_1$ relaxation measurements, and the Carr-Purcell-Meiboom-Gill (CPMG) pulse sequence was used for T$_2$ relaxation measurements. Data were fit to single or biexponential decay functions using the nonlinear least squares regression technique.

*Preliminary Evaluation of the Tolerability of Intratracheally Injected Polymer Lung Surfactants in Adult Mice*

In this study, 7-weeks old female BALB/C mice (purchased from Jackson Laboratory) were used. Prior to injection of polymer lung surfactants, mice were analgetized by injecting 80 $\mu$l of 0.75 mg/ml Prevail (VetOne) to the back of the mouse neck. Mice were then anesthetized using isoflurane at 2 l/min flow rate. Once mice were completely sedated, tracheotomy was performed to inject the polymer lung surfactant solution. Three different doses were tested: 0.64, 6.5 and 64 mg polymer per kg mouse body weight. The liquid injection volume was kept at 20 $\mu$l, which corresponded to 1.07 ml liquid per kg body weight. After polymer injection, mice were sutured, and GLUture (Abbott Laboratories) was applied to the surgery area. Mice were monitored by experienced animal technicians for indications of toxicity (weight loss and behavioral symptoms).

After day 14, mice were sacrificed, and major organs were collected, perfused with PBS, and fixed with 10 % formalin. For the preparation of lung tissue an additional step was applied: inflation of the excised lung with 10 % formalin prior to fixation. The collected organ tissues were sliced and stained with H&E for microscopy examination.

*Pressure- Volume Mechanics of Rabbit Fetus Lungs Following Administration of Polymer Lung Surfactants*

P-V mechanics of 27-day gestation New Zealand White Rabbit fetus lungs were tested *ex vivo* following administration of polymer lung surfactants. Fetuses were obtained by Cesarean section. Polymer lung surfactants (or Newfacten) were injected into the rabbit fetus lung by single intratracheal instillation of 1.5 ml liquid per kg body weight. Polymer lung surfactant doses tested were 6, 60 and 96 mg polymer per kg body weight, and the Newfacten dose was 60 mg/kg. After lung surfactant instillation, 10 minutes were waited before P-V analysis.

For the avoidance of doubt, reference to methods of treatment described herein are understood to relate to compositions for use in such methods as defined in the claims. Based on the above description, we can now disclose a method of treating pulmonary disorders, including infant, acute or adult respiratory distress syndromes, caused by deficiency of functional lung surfactant in mammals, including humans. The method includes administering to an animal or human subject a therapeutically effective amount of polymer lung surfactant composition. The polymer lung surfactant composition comprises an effective amount of a synthetic biocompatible or biodegradable amphiphilic homopolymer or copolymer whose monomers are selected from the group consisting of: ethylene glycol (EG), ethylene oxide (EO), vinyl alcohol (VA), alkyl oxazoline (AO), D,L-lactic acid or D,L-lactide (LA), glycolic acid or glycolide (GA), $\varepsilon$-caprolactone (CL), styrene (PS), alkyl methacrylate (AMA), and alkyl acrylate (AA).

Another method of treating pulmonary disorders, including infant, acute or adult respiratory distress syndromes, caused by deficiency of functional lung surfactant in mammals, including humans, wherein the method includes administering to an animal or human subject a synthetic block as a single therapeutic agent or in combination with other therapeutics.

Yet another method of method of treating pulmonary disorders, including infant, acute or adult respiratory distress syndromes, caused by deficiency of functional lung surfactant in mammals, including humans, includes administering to an animal or human subject a synthetic random copolymer to the subject as a single therapeutic agent or in combination with other therapeutics.

Another method of treating pulmonary disorders, including infant, acute or adult respiratory distress syndromes, caused by deficiency of functional lung surfactant in mammals, including humans, wherein the method includes administering to an animal or human subject a synthetic homopolymer to the subject as a single therapeutic agent or in combination with other therapeutics.

Yet another method of treating pulmonary disorders, including infant, acute or adult respiratory distress syndromes, caused by deficiency of functional lung surfactant in mammals, including humans, wherein the method includes administering to an animal or human subject a polymer lung surfactant composition comprising a poly(styrene-block-ethylene glycol) (PS-PEG) block copolymer.

Another method of treating pulmonary disorders, including infant, acute or adult respiratory distress syndromes, caused by deficiency of functional lung surfactant in mammals, including humans, wherein the method includes administering to an animal or human subject a polymer lung surfactant composition comprising a poly(tert-butyl methacrylate-block-ethylene glycol) (PtBMA-PEG) block copolymer.

Another method of treating pulmonary disorders, including infant, acute or adult respiratory distress syndromes, caused by deficiency of functional lung surfactant in mammals, including humans, wherein the method includes administering to an animal or human subject a polymer lung surfactant composition comprising a poly(D,L-lactic acid-block-ethylene glycol) (PLA-PEG) block copolymer.

A method of treating pulmonary disorders, including infant, acute or adult respiratory distress syndromes, caused by deficiency of functional lung surfactant in mammals, including humans, wherein the method includes administering to an animal or human subject, a polymer lung surfactant composition to the lungs of the animal or human subject in the form of an aqueous solution via endotracheal instillation.

A method of treating pulmonary disorders, including infant, acute or adult respiratory distress syndromes, caused by deficiency of functional lung surfactant in mammals, including humans, wherein the method includes including administering to an animal or human subject a polymer lung surfactant composition to the patient's lungs in the form of liquid drop or dry powder-type aerosols through application of continuous positive airway pressure.

Exemplary polymer lung surfactant composition used in above method of treatment have a formulation comprising about 0.02 - 20 wt.% amphiphilic block copolymers dispersed in micelle form in aqueous saline solution, wherein the amphiphilic block copolymer compound comprises a hydrophilic block (e.g., PEG) having an average molecular weight in the range between about 50 Da and about 500 kDa and a hydrophobic block (e.g., PS) having an average molecular weight in the range between about 50 Da and about 500 kDa.

It should be note that all the above described methods can be used in treating infant, acute or adult respiratory distress syndromes. Further, these methods can also be used in treating bronchopulmonary dysplasia.

Those skilled in the art will recognize that numerous modifications can be made to the specific implementations described above. The implementations should not be limited to the particular limitations described. Other implementations may be possible. In addition, several publications relevant to the disclosure are listed below and are cited herein.

**References**

1 Hogan, Brigid L. M. et al. Repair and Regeneration of the Respiratory System: Complexity, Plasticity, and Mechanisms of Lung Stem Cell Function. Cell Stem Cell 15, 123-138, doi:http://dx.doi.org/10.1016/j.stem.2014.07.012 (2014).

2 Notter, R. H. Lung Surfactants: Basic Science and Clinical Applications. (Taylor & Francis, 2000).

3 Wrobel, S. Bubbles, Babies and Biology: The Story of Surfactant. The FASEB Journal 18, 1624e, doi:10.1096/fj.04-2077bkt (2004).

4 Singh, G. K. & van Dyck, P. C. (U.S. Department of Health and Human Services, Health Resources and Services Administration, Maternal and Child Health Bureau, 2010).

5 Liggins, G. C. & Howie, R. N. A CONTROLLED TRIAL OF ANTEPARTUM GLUCOCORTICOID TREATMENT FOR PREVENTION OF THE RESPIRATORY DISTRESS SYNDROME IN PREMATURE INFANTS. Pediatrics 50, 515-525 (1972).

6 Clements, J. A. & Avery, M. E. Lung Surfactant and Neonatal Respiratory Distress Syndrome. American Journal of Respiratory and Critical Care Medicine 157, S59-S66, doi:10.1164/ajrccm.157.4.nhlb1-1 (1998).

7 Halliday, H. L. Surfactants: past, present and future. J Perinatol 28, S47-S56 (0000).

8 World Health Organization Essential Medicine List, <www.who.int/entity/selection_medicines/list/en/> (

9 Gregory, G. A., Kitterman , J. A., Phibbs , R. H., Tooley , W. H. & Hamilton , W. K. Treatment of the Idiopathic Respiratory-Distress Syndrome with Continuous Positive Airway Pressure. New England Journal of Medicine 284, 1333-1340, doi:doi:10.1056/NEJIM197106172842401 (1971).

10 Kamath, B. D., MacGuire, E. R., McClure, E. M., Goldenberg, R. L. & Jobe, A. H. Neonatal Mortality From Respiratory Distress Syndrome: Lessons for Low-Resource Countries. Pediatrics 127, 1139-1146, doi:10.1542/peds.2010-3212 (2011).

11 Vidyasagar, D., Velaphi, S. & Bhat, V. B. Surfactant Replacement Therapy in Developing Countries. Neonatology 99, 355-366 (2011).

12 Neumann, A. W., David, R. & Zuo, Y. Applied Surface Thermodynamics, Second Edition. (CRC Press, 2010).

13 and, T. E. W. & Conkright, J. J. Function of Surfactant Proteins B and C. Annual Review of Physiology 63, 555-578, doi:doi:10.1146/annurev.physiol.63.1.555 (2001).

14 Chu, J. et al. NEONATAL PULMONARY ISCHEMIA. Part I: Clinical and Physiological Studies 40, 709-782 (1967).

15 Robillard, E., Alarie, Y., Dagenais-Perusse, P., Baril, E. & Guilbeault, A. Microaerosol Administration of Synthetic β-γ-Dipalmitoyl-L-α-Lecithin in the Respiratory Distress Syndrome: A Preliminary Report. Canadian Medical Association Journal 90, 55-57 (1964).

16 Cochrane, C. G. et al. The efficacy and safety of KL4-surfactant in preterm infants with respiratory distress syndrome. American Journal of Respiratory and Critical Care Medicine 153, 404-410, doi:10.1164/ajrccm.153.1.8542150 (1996).

17 Wiswell, T. E. et al. Bronchopulmonary Segmental Lavage with Surfaxin (KL4-Surfactant) for Acute Respiratory Distress Syndrome. American Journal of Respiratory and Critical Care Medicine 160, 1188-1195, doi:10.1164/ajrccm.160.4.9808118 (1999).

18 Garner, S. S. & Cox, T. H. Lucinactant: New and Approved, But Is It an Improvement? The Journal of Pediatric Pharmacology and Therapeutics : JPPT 17, 206-210, doi:10.5863/1551-6776-17.3.206 (2012).

19 Moya, F. et al. One-Year Follow-up of Very Preterm Infants Who Received Lucinactant for Prevention of Respiratory Distress Syndrome: Results From 2 Multicenter Randomized, Controlled Trials. Pediatrics 119, e1361-e1370, doi:10.1542/peds.2006-0149 (2007).

20 Singh, N., Hawley, K. L. & Viswanathan, K. Efficacy of Porcine Versus Bovine Surfactants for Preterm Newborns With Respiratory Distress Syndrome: Systematic Review and Meta-analysis. Pediatrics 128, e1588-e1595, doi:10.1542/peds.2011-1395 (2011).

21 Kattwinkel, J. Synthetic Surfactants: The Search Goes on. Pediatrics 115, 1075-1076, doi:10.1542/peds.2005-0202 (2005).

22 Engle, W. A. & Stark, A. R. Surfactant-Replacement Therapy for Respiratory Distress Syndrome in the Preterm and Term Neonate: Congratulations and Corrections: In Reply. Pediatrics 121, 1291-1292, doi:10.1542/peds.2008-0910 (2008).

23 Engle, W. A. Surfactant-Replacement Therapy for Respiratory Distress in the Preterm and Term Neonate. Pediatrics 121, 419-432, doi:10.1542/peds.2007-3283 (2008).

24 Gdovin, J. M., Moya, F., Vishalpura, T. & Grogg, A. A Comparative Pharmacoeconomic Assessment of Two Surfactants for the Prevention of Respiratory Distress Syndrome. The Journal of Pediatric Pharmacology and Therapeutics : JPPT 11, 43-54, doi:10.5863/1551-6776-11.1.43 (2006).

25 Marsh, W., Smeeding, J., York, J. M., Ramanathan, R. & Sekar, K. A Cost Minimization Comparison of Two Surfactants-Beractant and Poractant alfa-Based Upon Prospectively Designed, Comparative Clinical Trial Data. The Journal of Pediatric Pharmacology and Therapeutics : JPPT 9, 117-125, doi:10.5863/1551-6776-9.2.117 (2004).

26 Dunn, S. E., Brindley, A., Davis, S. S., Davies, M. C. & Illum, L. Polystyrene-Poly (Ethylene Glycol) (PS-PEG2000) Particles as Model Systems for Site Specific Drug Delivery. 2. The Effect of PEG Surface Density on the in Vitro Cell Interaction and in VivoBiodistribution. Pharmaceutical Research 11, 1016-1022, doi:10.1023/a:1018939521589 (1994).

27 Kutscher, H. L. et al. Enhanced passive pulmonary targeting and retention of PEGylated rigid microparticles in rats. International Journal of Pharmaceutics 402, 64-71, doi:http://dx.doi.org/10.1016/j.ijpharm.2010.09.020 (2010).

28 Beroström, K. et al. Effects of branching and molecular weight of surface-bound poly(ethylene oxide) on protein rejection. Journal of Biomaterials Science, Polymer Edition 6, 123-132, doi:10.1163/156856294X00257 (1995).

29 Meng, F., Engbers, G. H. M., Gessner, A., Miiller, R. H. & Feijen, J. Pegylated polystyrene particles as a model system for artificial cells. Journal of Biomedical Materials Research Part A 70A, 97-106, doi:10.1002/jbm.a.30068 (2004).

30 Laan, A. C. et al. Radiolabeling polymeric micelles for in vivo evaluation: a novel, fast, and facile method. EJNMMI Research 6, 12, doi:10.1186/s13550-016-0167-x (2016).

31 Pressly, E. D. et al. Structural Effects on the Biodistribution and Positron Emission Tomography (PET) Imaging of Well-Defined 64Cu-Labeled Nanoparticles Comprised of Amphiphilic Block Graft Copolymers. Biomacromolecules 8, 3126-3134, doi:10.1021/bm700541e (2007).

32 Zhang, L. & Eisenberg, A. Multiple morphologies of "crew-cut" aggregates of polystyrene-b-poly(acrylic acid) block copolymers. Science 268, 1728+ (1995).

33 Zhang, L. & Eisenberg, A. Multiple Morphologies and Characteristics of "Crew-Cut" Micelle-like Aggregates of Polystyrene-b-poly(acrylic acid) Diblock Copolymers in Aqueous Solutions. Journal of the American Chemical Society 118, 3168-3181, doi:10.1021/ja953709s (1996).

34 Park, H.-W. et al. Study of the Air-Water Interfacial Properties of Biodegradable Polyesters and Their Block Copolymers with Poly(ethylene glycol). Langmuir 28, 11555-11566, doi:10.1021/1a300810q (2012).

35 Kim, H. C., Lee, H., Khetan, J. & Won, Y.-Y. Surface Mechanical and Rheological Behaviors of Biocompatible Poly ((D, L-lactic acid-ran-glycolic acid)-block-ethylene glycol)(PLGA-PEG) and Poly ((D, L-lactic acid-ran-glycolic acid-ran-ε-caprolactone)-block-ethylene glycol)(PLGACL-PEG) Block Copolymers at the Air-Water Interface. Langmuir 31, 13821-13833 (2015).

36 Kim, H. C. et al. Humidity-Dependent Compression-Induced Glass Transition of the Air-Water Interfacial Langmuir Films of Poly (D, L-lactic acid-ran-glycolic acid)(PLGA). Soft Matter 11, 5666-5677 (2015).

37 Gavranovic, G. T., Deutsch, J. M. & Fuller, G. G. Two-Dimensional Melts: Polymer Chains at the Air-Water Interface. Macromolecules 38, 6672-6679, doi:10.1021/ma050061n (2005).

38 Gavranovic, G. T. et al. Effects of Temperature and Chemical Modification on Polymer Langmuir Films. The Journal of Physical Chemistry B 110, 22285-22290, doi:10.1021/jp063396v (2006).

39 Polymer Interface and Adhesion. (Taylor & Francis, 1982).

40 Brandrup, J., Immergut, E. H. & Grulke, E. A. Polymer Handbook. (Wiley, 1999).

41 Cheyne, R. B. & Moffitt, M. G. Novel Two-Dimensional "Ring and Chain" Morphologies in Langmuir-Blodgett Monolayers of PS-b-PEO Block Copolymers: Effect of Spreading Solution Concentration on Self-Assembly at the Air-Water Interface. Langmuir 21, 5453-5460, doi:10.1021/1a0503707 (2005).

42 Francis, R. et al. Synthesis and Surface Properties of Amphiphilic Star-Shaped and Dendrimer-like Copolymers Based on Polystyrene Core and Poly(ethylene oxide) Corona. Macromolecules 36, 8253-8259, doi:10.1021/ma030258k (2003).

43 Gonçalves da Silva, A. M., Filipe, E. J. M., d'Oliveira, J. M. R. & Martinho, J. M. G. Interfacial Behavior of Poly(styrene)-Poly(ethylene oxide) Diblock Copolymer Monolayers at the Air-Water Interface. Hydrophilic Block Chain Length and Temperature Influence. Langmuir 12, 6547-6553, doi:10.1021/1a960604+ (1996).

44 Gonçalves da Silva, A. M., Simões Gamboa, A. L. & Martinho, J. M. G. Aggregation of Poly(styrene)-Poly(ethylene oxide) Diblock Copolymer Monolayers at the Air-Water Interface. Langmuir 14, 5327-5330, doi:10.1021/1a980101z (1998).

45 Fauré, M. C., Bassereau, P., Lee, L. T., Menelle, A. & Lheveder, C. Phase Transitions in Monolayers of PS-PEO Copolymer at the Air-Water Interface. Macromolecules 32, 8538-8550, doi:10.1021/ma9900840 (1999).

46 Peleshanko, S., Jeong, J., Gunawidjaja, R. & Tsukruk, V. V. Amphiphilic Heteroarm PEO-b-PSm Star Polymers at the Air-Water Interface: Aggregation and Surface Morphology. Macromolecules 37, 6511-6522, doi:10.1021/ma0493170 (2004).

47 Cheyne, R. B. & Moffitt, M. G. Self-Assembly of Polystyrene-block-Poly(Ethylene Oxide) Copolymers at the Air-Water Interface: Is Dewetting the Genesis of Surface Aggregate Formation? Langmuir 22, 8387-8396, doi:10.1021/1a061953z (2006).

48 Levitt, M. H. Spin Dynamics: Basics of Nuclear Magnetic Resonance. (Wiley, 2001).

49 Ries, M. E., Klein, P. G., Brereton, M. G. & Ward, I. M. Proton NMR Study of Rouse Dynamics and Ideal Glass Transition Temperature of Poly(ethylene oxide) LiCF3SO3 Complexes. Macromolecules 31, 4950-4956, doi:10.1021/ma971860b (1998).

50 Zheng, Y. et al. Directly Resolved Core-Corona Structure of Block Copolymer Micelles by Cryo-Transmission Electron Microscopy. The Journal of Physical Chemistry B 103, 10331-10334, doi:10.1021/jp9923264 (1999).

51 Won, Y.-Y., Davis, H. T., Bates, F. S., Agamalian, M. & Wignall, G. D. Segment Distribution of the Micellar Brushes of Poly(ethylene oxide) via Small-Angle Neutron Scattering. The Journal of Physical Chemistry B 104, 7134-7143, doi:10.1021/jp000457v (2000).

52 Nakos, G., Kitsiouli, E. I., Tsangaris, I. & Lekka, M. E. Bronchoalveolar lavage fluid characteristics of early intermediate and late phases of ARDS. Intensive Care Medicine 24, 296-303, doi:10.1007/s001340050571 (1998).

53 Spragg, R. G. in Pathophysiology of Shock, Sepsis, and Organ Failure (eds Günther Schlag & Heinz Redl) 747-756 (Springer Berlin Heidelberg, 1993).

54 Spragg , R. G. et al. Effect of Recombinant Surfactant Protein C-Based Surfactant on the Acute Respiratory Distress Syndrome. New England Journal of Medicine 351, 884-892, doi:doi:10.1056/NEJMoa033181 (2004).

55 Gregory, T. J. et al. Bovine surfactant therapy for patients with acute respiratory distress syndrome. American Journal of Respiratory and Critical Care Medicine 155, 1309-1315, doi:10.1164/ajrccm.155.4.9105072 (1997).

56 Filoche, M., Tai, C.-F. & Grotberg, J. B. Three-dimensional model of surfactant replacement therapy. Proceedings of the National Academy of Sciences 112, 9287-9292, doi:10.1073/pnas.1504025112 (2015).

57 Grotberg, J. B., Filoche, M., Willson, D. F., Raghavendran, K. & Notter, R. H. Did Reduced Alveolar Delivery of Surfactant Contribute to Negative Results in Adults with Acute Respiratory Distress Syndrome? American Journal of Respiratory and Critical Care Medicine 195, 538-540, doi:10.1164/rccm.201607-1401LE (2017).

58 King, D. M., Wang, Z., Palmer, H. J., Holm, B. A. & Notter, R. H. Bulk shear viscosities of endogenous and exogenous lung surfactants. American Journal of Physiology - Lung Cellular and Molecular Physiology 282, L277-L284, doi:10.1152/ajplung.00199.2001 (2002).

59 King, D. M. et al. Concentration-dependent, temperature-dependent non-Newtonian viscosity of lung surfactant dispersions. Chemistry and Physics of Lipids 112, 11-19, doi:https://doi.org/10.1016/S0009-3084(01)00150-5 (2001).

60 Berggren, E. et al. Pilot study of nebulized surfactant therapy for neonatal respiratory distress syndrome. Acta Pædiatrica 89, 460-464, doi:10.1111/j.1651-2227.2000.tb00084.x (2000).

61 Lewis, J. F., Ikegami, M., Jobe, A. H. & Tabor, B. Aerosolized surfactant treatment of preterm lambs. Journal of Applied Physiology 70, 869-876 (1991).

62 Ellyett, K. M., Broadbent, R. S., Fawcett, E. R. & Campbell, A. J. Surfactant Aerosol Treatment of Respiratory Distress Syndrome in the Spontaneously Breathing Premature Rabbit. Pediatr Res 39, 953-957 (1996).

63 Dijk, P. H., Heikamp, A. & Oetomo, S. B. Surfactant Nebulization versus Instillation during High Frequency Ventilation in Surfactant-Deficient Rabbits. Pediatr Res 44, 699-704 (1998).

64 Edwards, D. A., Ben-Jebria, A. & Langer, R. Recent advances in pulmonary drug delivery using large, porous inhaled particles. Journal of Applied Physiology 85, 379-385 (1998).

65 Sung, J. C., Pulliam, B. L. & Edwards, D. A. Nanoparticles for drug delivery to the lungs. Trends in Biotechnology 25, 563-570, doi:http://dx.doi.org/10.1016/j.tibtech.2007.09.005 (2007).

66 Tsapis, N., Bennett, D., Jackson, B., Weitz, D. A. & Edwards, D. A. Trojan particles: Large porous carriers of nanoparticles for drug delivery. Proceedings of the National Academy of Sciences 99, 12001-12005, doi:10.1073/pnas.182233999 (2002).

67 Ungaro, F. et al. Dry powders based on PLGA nanoparticles for pulmonary delivery of antibiotics: Modulation of encapsulation efficiency, release rate and lung deposition pattern by hydrophilic polymers. Journal of Controlled Release 157, 149-159, doi:http://dx doi.org/10.1016/j.jconrel.2011.08.010 (2012).

68 Yamamoto, H., Kuno, Y., Sugimoto, S., Takeuchi, H. & Kawashima, Y. Surface-modified PLGA nanosphere with

EP 3 496 732 B1

chitosan improved pulmonary delivery of calcitonin by mucoadhesion and opening of the intercellular tight junctions. Journal of Controlled Release 102, 373-381, doi:http://dx.doi.org/10.1016/j.jconrel.2004.10.010 (2005).

69 Menon, J. U. et al. Polymeric Nanoparticles for Pulmonary Protein and DNA Delivery. Acta biomaterialia 10, 2643-2652, doi:10.1016/j.actbio.2014.01.033 (2014).

70 Neises, B. & Steglich, W. Simple Method for the Esterification of Carboxylic Acids. Angewandte Chemie International Edition in English 17, 522-524, doi:10.1002/anie.197805221 (1978).

**Claims**

1. A composition comprising a synthetic polymer material for use in a method of treating pulmonary disorders selected from infant, acute or adult respiratory distress syndromes caused by deficiency of functional lung surfactant in mammals, the method comprising: administering to an animal or human subject the composition, wherein the synthetic polymer material is formulated into an aqueous micelle solution and wherein the synthetic polymer material is a block co-polymer selected from:

   poly(tert-butyl methacrylate-block-ethylene glycol); and
   poly(styrene-block-ethylene glycol).

2. A composition comprising a synthetic polymer material for use in a method of treating bronchopulmonary dysplasia, wherein the synthetic polymer material is formulated into an aqueous micelle solution and wherein the synthetic polymer material is a block co-polymer selected from:

   poly(tert-butyl methacrylate-block-ethylene glycol); and
   poly(styrene-block-ethylene glycol).

3. The composition for use of claim 1 or claim 2, wherein the composition comprises poly(styrene-block-ethylene glycol) block copolymer.

4. The composition for use of claim 1 or claim 2, wherein the composition comprises poly(tert-butyl methacrylate-block-ethylene glycol) block copolymer.

5. The composition for use of claim 1 or claim 2 wherein the method comprising:
   administering to an animal or human subject the composition to the lungs of the animal or human subject in the form of an aqueous solution via endotracheal instillation.

6. The composition for use of claim 1 or claim 2 wherein the method comprising:
   administering to an animal or human subject the composition to the lungs of the animal or human in the form of a liquid drop or dry powder-type aerosols through application of positive pressure, for example continuous positive airway pressure.

7. The composition for use of claim 1 or claim 2 wherein the method comprising:
   administering to an animal or human subject the composition in combination with other therapeutics.

8. The composition for use of claim 1 or claim 2 comprising about 0.02 ~ 20 wt.% of amphiphilic block copolymers dispersed in micelle form in an aqueous or aqueous saline solution, wherein said amphiphilic polymer comprises a plurality of hydrophilic blocks having an average molecular weight in the range of about 50 Da ~ 500 kDa and a plurality of hydrophobic blocks having an average molecular weight in the range of about 50 Da ~ 500 kDa, wherein said amphiphilic polymer is poly(tert-butyl methacrylate-block-ethylene glycol) (PtBMA-PEG) block copolymer.

9. The composition for use of claim 1 or claim 2, comprising about 0.02 - 20 wt.% amphiphilic block copolymers dispersed in micelle form in aqueous or aqueous saline solution, wherein the amphiphilic block copolymer compound comprises a plurality of hydrophilic blocks comprising polyethyl glycol having an average molecular weight in the range between about 50 Da and about 500 kDa and a plurality of hydrophobic blocks comprising polystyrene having an average molecular weight in the range between about 50 Da and about 500 kDa, wherein said amphiphilic polymer is poly(styrene-block-ethylene glycol) (PS-b-PEG) block copolymer.

10. The composition for use of any preceding claim wherein the synthetic polymer material is aerosolized in liquid or powder form.

**Patentansprüche**

1. Zusammensetzung, umfassend ein synthetisches Polymermaterial zur Verwendung in einem Verfahren zum Behandeln von Lungenerkrankungen, ausgewählt aus Atemnotsyndrom des Neugeborenen, akutem Atemnotsyndrom oder Atemnotsyndrom des Erwachsenen, die durch einen Mangel an funktionellem Lungen-Surfactant verursacht werden, bei Säugern, wobei das Verfahren Folgendes umfasst: Verabreichen der Zusammensetzung an ein tierisches oder menschliches Subjekt, wobei das synthetische Polymermaterial zu einer wässrigen Mizellenlösung formuliert ist und wobei das synthetische Polymermaterial ein Blockcopolymer ist, ausgewählt aus:

   Poly(tert-butylmethacrylat-Block-Ethylenglycol); und
   Poly(styrol-Block-Ethylenglycol).

2. Zusammensetzung, umfassend ein synthetisches Polymermaterial zur Verwendung in einem Verfahren zum Behandeln von bronchopulmonaler Dysplasie, wobei das synthetische Polymermaterial zu einer wässrigen Mizellenlösung formuliert ist und wobei das synthetische Polymermaterial ein Blockcopolymer ist, ausgewählt aus:

   Poly(tert-butylmethacrylat-Block-Ethylenglycol); und
   Poly(styrol-Block-Ethylenglycol).

3. Zusammensetzung zur Verwendung nach Anspruch 1 oder Anspruch 2, wobei die Zusammensetzung Poly(styrol-Block-Ethylenglykol)-Blockcopolymer umfasst.

4. Zusammensetzung zur Verwendung nach Anspruch 1 oder Anspruch 2, wobei die Zusammensetzung Poly(tert-butylmethacrylat-Block-Ethylenglykol)-Blockcopolymer umfasst.

5. Zusammensetzung zur Verwendung nach Anspruch 1 oder Anspruch 2, wobei das Verfahren Folgendes umfasst: Verabreichen der Zusammensetzung an die Lungen des tierischen oder menschlichen Subjekts in Form einer wässrigen Lösung durch endotracheale Instillation an ein tierisches oder menschliches Subjekt.

6. Zusammensetzung zur Verwendung nach Anspruch 1 oder Anspruch 2, wobei das Verfahren Folgendes umfasst: Verabreichen der Zusammensetzung an die Lunge des Tieres oder Menschen in Form eines Flüssigkeitstropfens oder trockener pulverförmiger Aerosole an ein tierisches oder menschliches Subjekt durch Anlegen eines positiven Drucks, zum Beispiel kontinuierlichen positiven Atemwegsdrucks.

7. Zusammensetzung zur Verwendung nach Anspruch 1 oder Anspruch 2, wobei das Verfahren Folgendes umfasst: Verabreichen der Zusammensetzung in Kombination mit anderen Therapeutika an ein tierisches oder menschliches Subjekt.

8. Zusammensetzung zur Verwendung nach Anspruch 1 oder Anspruch 2, umfassend etwa 0,02 ~ 20 Gew.-% amphiphile Blockcopolymere, die in Mizellenform in einer wässrigen oder wässrigen Salzlösung dispergiert sind, wobei das amphiphile Polymer eine Vielzahl von hydrophilen Blöcken mit einem durchschnittlichen Molekulargewicht im Bereich von etwa 50 Da ~ 500 kDa und eine Vielzahl von hydrophoben Blöcken mit einem durchschnittlichen Molekulargewicht im Bereich von etwa 50 Da ~ 500 kDa umfasst, wobei das amphiphile Polymer Poly(tert-butylmethacrylat-Block-Ethylenglykol)(PtBMA-PEG)-Blockcopolymer ist.

9. Zusammensetzung zur Verwendung nach Anspruch 1 oder Anspruch 2, umfassend etwa 0,02 - 20 Gew.-% amphiphile Blockcopolymere, die in Mizellenform in wässriger oder wässriger Salzlösung dispergiert sind, wobei die amphiphile Blockcopolymerverbindung eine Vielzahl von hydrophilen Blöcken, die Polyethylenglykol mit einem durchschnittlichen Molekulargewicht im Bereich zwischen etwa 50 Da und etwa 500 kDa umfassen, und eine Vielzahl von hydrophoben Blöcken umfasst, die Polystyrol mit einem durchschnittlichen Molekulargewicht im Bereich zwischen etwa 50 Da und etwa 500 kDa umfassen, wobei das amphiphile Polymer Poly(styrol-Block-Ethylenglykol)(PS-b-PEG)-Blockcopolymer ist.

10. Zusammensetzung zur Verwendung nach einem vorhergehenden Anspruch, wobei das synthetische Polymermaterial in flüssiger oder Pulverform aerosoliert wird.

**Revendications**

1. Composition comprenant un matériau polymère synthétique destinée à être utilisée dans un procédé de traitement de troubles pulmonaires choisis parmi les syndromes de détresse respiratoire infantile, aiguë ou de l'adulte causés par une déficience en tensioactif pulmonaire fonctionnel chez les mammifères, le procédé comprenant : l'administration de la composition à un sujet animal ou humain, dans laquelle le matériau polymère synthétique est formulé sous forme d'une solution micellaire aqueuse et dans laquelle le matériau polymère synthétique est un copolymère bloc choisi parmi :

   le poly(méthacrylate de tert-butyle-bloc-éthylène glycol) ; et
   le poly(styrène-bloc-éthylène glycol).

2. Composition comprenant un matériau polymère synthétique destinée à être utilisée dans un procédé de traitement de la dysplasie bronchopulmonaire, dans laquelle le matériau polymère synthétique est formulé sous forme d'une solution micellaire aqueuse et dans laquelle le matériau polymère synthétique est un copolymère bloc choisi parmi :

   le poly(méthacrylate de tert-butyle-bloc-éthylène glycol) ; et
   le poly(styrène-bloc-éthylène glycol).

3. Composition destinée à être utilisée selon la revendication 1 ou la revendication 2, dans laquelle la composition comprend un copolymère bloc poly(styrène-bloc-éthylène glycol).

4. Composition destinée à être utilisée selon la revendication 1 ou la revendication 2, dans laquelle la composition comprend un copolymère bloc poly(méthacrylate de tert-butyle-bloc-éthylène glycol).

5. Composition destinée à être utilisée selon la revendication 1 ou la revendication 2, dans laquelle le procédé comprend :
   l'administration, à un sujet animal ou humain, de la composition aux poumons du sujet animal ou humain sous la forme d'une solution aqueuse par instillation endotrachéale.

6. Composition destinée à être utilisée selon la revendication 1 ou la revendication 2, dans laquelle le procédé comprend :
   l'administration, à un sujet animal ou humain, de la composition aux poumons de l'animal ou de l'humain sous forme de gouttes liquides ou d'aérosols de type poudre sèche par application d'une pression positive, par exemple une pression positive continue dans les voies respiratoires.

7. Composition destinée à être utilisée selon la revendication 1 ou la revendication 2, dans laquelle le procédé comprend :
   l'administration, à un sujet animal ou humain, de la composition en combinaison avec d'autres agents thérapeutiques.

8. Composition destinée à être utilisée selon la revendication 1 ou la revendication 2, comprenant environ 0,02 ~ 20 % en poids de copolymères blocs amphiphiles dispersés sous forme de micelles dans une solution aqueuse ou aqueuse saline, dans laquelle ledit polymère amphiphile comprend une pluralité de blocs hydrophiles présentant un poids moléculaire moyen compris dans la plage d'environ 50 Da ~ 500 kDa et une pluralité de blocs hydrophobes présentant un poids moléculaire moyen compris dans la plage d'environ 50 Da ~ 500 kDa, dans laquelle ledit polymère amphiphile est un copolymère bloc poly(méthacrylate de tert-butyle-bloc-éthylène glycol) (PtBMA-PEG).

9. Composition destinée à être utilisée selon la revendication 1 ou la revendication 2, comprenant environ 0,02 à 20 % en poids de copolymères blocs amphiphiles dispersés sous forme de micelles dans une solution aqueuse ou aqueuse saline, dans laquelle le composé copolymère bloc amphiphile comprend une pluralité de blocs hydrophiles comprenant du polyéthylglycol présentant un poids moléculaire moyen compris dans la plage d'environ 50 Da à environ 500 kDa et une pluralité de blocs hydrophobes comprenant du polystyrène présentant un poids moléculaire moyen compris dans la plage d'environ 50 Da à environ 500 kDa, dans laquelle ledit polymère amphiphile est un copolymère bloc poly(styrène-bloc-éthylène glycol) (PS-b-PEG).

10. Composition destinée à être utilisée selon une quelconque revendication précédente, dans laquelle le matériau polymère synthétique est aérosolisé sous forme liquide ou sous forme de poudre.

FIGURE 1

FIGURE 2

FIGURE 3

Poly(lactic acid-co-glycolic acid-
block-ethylene glycol)
**PLGA-PEG**

Poly(lactic acid-co-glycolic acid-co-ε-caprolactone-
block-ethylene glycol)
**PLGACL-PEG**

Poly(tert-butyl methacrylate-block-
ethylene glycol)
**PtBMA-PEG**

Poly(styrene-block-ethylene glycol)
**PS-PEG**

FIGURE 4

FIGURE 5

FIGURE 6

FIGURE 7

FIGURE 8

FIGURE 9

FIGURE 10

FIGURE 11

FIGURE 12

FIGURE 13

FIGURE 14

FIGURE 15

FIGURE 16

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2013004453 A1
- CN 101804021 A
- WO 9710849 A1
- US 8529917 B2

**Non-patent literature cited in the description**

- **PARK et al.** *Langmuir*, 2012, vol. 28 (31), 11555-11566
- **KIM et al.** *Langmuir*, 2015, vol. 31 (51), 13821-13833
- **COX et al.** *Langmuir*, 1999, vol. 15 (22), 7714-7718
- **MALZERT et al.** *Langmuir*, 2001, vol. 17 (25), 7837-7841
- **RANGER et al.** *Journal of Polymer Science Part A: Polymer Chemistry*, 2001, vol. 39 (22), 3861-3874
- **SHIN et al.** *Mol. Pharmaceutics*, 2011, vol. 8, 4, 1257-1265
- **MENON et al.** *Acta Biomaterialia*, 2014, vol. 10 (6), 2643-2652
- **HENNING et al.** *Journal of Aerosol Medicine and Pulmonary Drug Delivery*, 2010, vol. 23 (4), 233-241
- **BENAGLIA, M. et al.** Universal (Switchable) RAFT Agents. *Journal of the American Chemical Society*, 2009, vol. 131, 6914-6915
- **ZHANG, L.** ; **EISENBERG, A.** Multiple Morphologies and Characteristics of "Crew-Cut" Micelle-like Aggregates of Polystyrene-b-poly(acrylic acid) Diblock Copolymers in Aqueous Solutions. *Journal of the American Chemical Society*, 1996, vol. 118, 3168-3181
- **HOGAN, BRIGID L. M. et al.** Repair and Regeneration of the Respiratory System: Complexity, Plasticity, and Mechanisms of Lung Stem Cell Function.. *Cell Stem Cell*, 2014, vol. 15, 123-138, http://dx.doi.org/10.1016/j.stem.2014.07.012
- **NOTTER, R. H.** Lung Surfactants: Basic Science and Clinical Applications. Taylor & Francis, 2000
- **WROBEL, S. BUBBLES**. Babies and Biology: The Story of Surfactant. *The FASEB Journal*, 2004, vol. 18, 1624e
- **LIGGINS, G. C.** ; **HOWIE, R. N.** A CONTROLLED TRIAL OF ANTEPARTUM GLUCOCORTICOID TREATMENT FOR PREVENTION OF THE RESPIRATORY DISTRESS SYNDROME IN PREMATURE INFANTS. *Pediatrics*, 1972, vol. 50, 515-525
- **CLEMENTS, J. A.** ; **AVERY, M. E.** Lung Surfactant and Neonatal Respiratory Distress Syndrome.. *American Journal of Respiratory and Critical Care Medicine*, 1998, vol. 157, S59-S66
- **HALLIDAY, H. L.** Surfactants: past, present and future.. *J Perinatol*, vol. 28, S47-S56
- *World Health Organization Essential Medicine List*, www.who.int/entity/selection_medicines/list/en/
- **GREGORY, G. A.** ; **KITTERMAN, J. A.** ; **PHIBBS, R. H.** ; **TOOLEY, W. H.** ; **HAMILTON, W. K.** Treatment of the Idiopathic Respiratory-Distress Syndrome with Continuous Positive Airway Pressure. *New England Journal of Medicine*, 1971, vol. 284, 1333-1340
- **KAMATH, B. D.** ; **MACGUIRE, E. R.** ; **MCCLURE, E. M.** ; **GOLDENBERG, R. L.** ; **JOBE, A. H.** Neonatal Mortality From Respiratory Distress Syndrome: Lessons for Low-Resource Countries.. *Pediatrics*, 2011, vol. 127, 1139-1146
- **VIDYASAGAR, D.** ; **VELAPHI, S.** ; **BHAT, V. B.** Surfactant Replacement Therapy in Developing Countries.. *Neonatology*, 2011, vol. 99, 355-366
- **NEUMANN, A. W.** ; **DAVID, R.** ; **ZUO, Y.** Applied Surface Thermodynamics. CRC Press, 2010
- **T. E. W.** ; **CONKRIGHT, J. J.** Function of Surfactant Proteins B and C. *Annual Review of Physiology*, 2001, vol. 63, 555-578
- **CHU, J. et al.** NEONATAL PULMONARY ISCHEMIA. *Part I: Clinical and Physiological Studies*, 1967, vol. 40, 709-782
- **ROBILLARD, E.** ; **ALARIE, Y.** ; **DAGENAIS-PERUSSE, P.** ; **BARIL, E.** ; **GUILBEAULT, A.** Microaerosol Administration of Synthetic β-γ-Dipalmitoyl-L-α-Lecithin in the Respiratory Distress Syndrome: A Preliminary Report. *Canadian Medical Association Journal*, 1964, vol. 90, 55-57
- **COCHRANE, C. G. et al.** The efficacy and safety of KL4-surfactant in preterm infants with respiratory distress syndrome.. *American Journal of Respiratory and Critical Care Medicine*, 1996, vol. 153, 404-410

- **WISWELL, T. E. et al.** Bronchopulmonary Segmental Lavage with Surfaxin (KL4-Surfactant) for Acute Respiratory Distress Syndrome.. *American Journal of Respiratory and Critical Care Medicine*, 1999, vol. 160, 1188-1195
- **GARNER, S. S.** ; **COX, T. H.** Lucinactant: New and Approved, But Is It an Improvement?. *The Journal of Pediatric Pharmacology and Therapeutics : JPPT*, 2012, vol. 17, 206-210
- **MOYA, F. et al.** One-Year Follow-up of Very Preterm Infants Who Received Lucinactant for Prevention of Respiratory Distress Syndrome: Results From 2 Multicenter Randomized, Controlled Trials. *Pediatrics*, 2007, vol. 119, e1361-e1370
- **SINGH, N.** ; **HAWLEY, K. L.** ; **VISWANATHAN, K.** Efficacy of Porcine Versus Bovine Surfactants for Preterm Newborns With Respiratory Distress Syndrome: Systematic Review and Meta-analysis.. *Pediatrics*, 2011, vol. 128, e1588-e1595
- **KATTWINKEL, J.** Synthetic Surfactants: The Search Goes on.. *Pediatrics*, 2005, vol. 115, 1075-1076
- **ENGLE, W. A.** ; **STARK, A. R.** Surfactant-Replacement Therapy for Respiratory Distress Syndrome in the Preterm and Term Neonate: Congratulations and Corrections: In Reply. *Pediatrics*, 2008, vol. 121, 1291-1292
- **ENGLE, W. A.** Surfactant-Replacement Therapy for Respiratory Distress in the Preterm and Term Neonate. *Pediatrics*, 2008, vol. 121, 419-432
- **GDOVIN, J. M.** ; **MOYA, F.** ; **VISHALPURA, T.** ; **GROGG, A.** A Comparative Pharmacoeconomic Assessment of Two Surfactants for the Prevention of Respiratory Distress Syndrome.. *The Journal of Pediatric Pharmacology and Therapeutics : JPPT*, 2006, vol. 11, 43-54
- **MARSH, W.** ; **SMEEDING, J.** ; **YORK, J. M.** ; **RAMANATHAN, R.** ; **SEKAR, K.** A Cost Minimization Comparison of Two Surfactants-Beractant and Poractant alfa-Based Upon Prospectively Designed, Comparative Clinical Trial Data.. *The Journal of Pediatric Pharmacology and Therapeutics : JPPT*, 2004, vol. 9, 117-125
- **DUNN, S. E.** ; **BRINDLEY, A.** ; **DAVIS, S. S.** ; **DAVIES, M. C.** ; **ILLUM, L.** Polystyrene-Poly (Ethylene Glycol) (PS-PEG2000) Particles as Model Systems for Site Specific Drug Delivery. 2. The Effect of PEG Surface Density on the in Vitro Cell Interaction and in VivoBiodistribution.. *Pharmaceutical Research*, 1994, vol. 11, 1016-1022
- **KUTSCHER, H. L. et al.** Enhanced passive pulmonary targeting and retention of PEGylated rigid microparticles in rats.. *International Journal of Pharmaceutics*, 2010, vol. 402, 64-71, http://dx.doi.org/10.1016/j.ijpharm.2010.09.020
- **BEROSTRÖM, K. et al.** Effects of branching and molecular weight of surface-bound poly(ethylene oxide) on protein rejection.. *Journal of Biomaterials Science, Polymer Edition*, 1995, vol. 6, 123-132
- **MENG, F.** ; **ENGBERS, G. H. M.** ; **GESSNER, A.** ; **MIILLER, R. H.** ; **FEIJEN, J.** Pegylated polystyrene particles as a model system for artificial cells.. *Journal of Biomedical Materials Research Part A*, 2004, vol. 70A, 97-106
- **LAAN, A. C. et al.** Radiolabeling polymeric micelles for in vivo evaluation: a novel, fast, and facile method.. *EJNMMI Research*, 2016, vol. 6, 12
- **PRESSLY, E. D. et al.** Structural Effects on the Biodistribution and Positron Emission Tomography (PET) Imaging of Well-Defined 64Cu-Labeled Nanoparticles Comprised of Amphiphilic Block Graft Copolymers.. *Biomacromolecules*, 2007, vol. 8, 3126-3134
- **ZHANG, L.** ; **EISENBERG, A.** Multiple morphologies of "crew-cut" aggregates of polystyrene-b-poly(acrylic acid) block copolymers.. *Science*, 1995, vol. 268, 1728
- **ZHANG, L.** ; **EISENBERG, A.** Multiple Morphologies and Characteristics of "Crew-Cut" Micelle-like Aggregates of Polystyrene-b-poly(acrylic acid) Diblock Copolymers in Aqueous Solutions.. *Journal of the American Chemical Society*, 1996, vol. 118, 3168-3181
- **PARK, H.-W. et al.** Study of the Air-Water Interfacial Properties of Biodegradable Polyesters and Their Block Copolymers with Poly(ethylene glycol).. *Langmuir*, 2012, vol. 28, 11555-11566
- **KIM, H. C.** ; **LEE, H.** ; **KHETAN, J.** ; **WON, Y.-Y.** Surface Mechanical and Rheological Behaviors of Biocompatible Poly ((D, L-lactic acid-ran-glycolic acid)-block-ethylene glycol)(PLGA-PEG) and Poly ((D, L-lactic acid-ran-glycolic acid-ran-ε-caprolactone)-block-ethylene glycol)(PLGACL-PEG) Block Copolymers at the Air-Water Interface. *Langmuir*, 2015, vol. 31, 13821-13833
- **KIM, H. C. et al.** Humidity-Dependent Compression-Induced Glass Transition of the Air-Water Interfacial Langmuir Films of Poly (D, L-lactic acid-ran-glycolic acid)(PLGA). *Soft Matter*, 2015, vol. 11, 5666-5677
- **GAVRANOVIC, G. T.** ; **DEUTSCH, J. M.** ; **FULLER, G. G.** Two-Dimensional Melts: Polymer Chains at the Air-Water Interface.. *Macromolecules*, 2005, vol. 38, 6672-6679
- **GAVRANOVIC, G. T. et al.** Effects of Temperature and Chemical Modification on Polymer Langmuir Films.. *The Journal of Physical Chemistry B*, 2006, vol. 110, 22285-22290
- Polymer Interface and Adhesion. Taylor & Francis, 1982
- **BRANDRUP, J.** ; **IMMERGUT, E. H.** ; **GRULKE, E. A.** Polymer Handbook. Wiley, 1999

- **CHEYNE, R. B.** ; **MOFFITT, M. G.** Novel Two-Dimensional "Ring and Chain" Morphologies in Langmuir-Blodgett Monolayers of PS-b-PEO Block Copolymers: Effect of Spreading Solution Concentration on Self-Assembly at the Air-Water Interface. *Langmuir*, 2005, vol. 21, 5453-5460
- **FRANCIS, R. et al.** Synthesis and Surface Properties of Amphiphilic Star-Shaped and Dendrimer-like Copolymers Based on Polystyrene Core and Poly(ethylene oxide) Corona.. *Macromolecules*, 2003, vol. 36, 8253-8259
- **GONÇALVES DA SILVA, A. M.** ; **FILIPE, E. J. M.** ; **D'OLIVEIRA, J. M. R.** ; **MARTINHO, J. M. G.** Interfacial Behavior of Poly(styrene)-Poly(ethylene oxide) Diblock Copolymer Monolayers at the Air-Water Interface. Hydrophilic Block Chain Length and Temperature Influence.. *Langmuir*, 1996, vol. 12, 6547-6553
- **GONÇALVES DA SILVA, A. M.** ; **SIMÕES GAMBOA, A. L.** ; **MARTINHO, J. M. G.** Aggregation of Poly(styrene)-Poly(ethylene oxide) Diblock Copolymer Monolayers at the Air-Water Interface.. *Langmuir*, 1998, vol. 14, 5327-5330
- **FAURÉ, M. C.** ; **BASSEREAU, P.** ; **LEE, L. T.** ; **MENELLE, A.** ; **LHEVEDER, C.** Phase Transitions in Monolayers of PS-PEO Copolymer at the Air-Water Interface.. *Macromolecules*, 1999, vol. 32, 8538-8550
- **PELESHANKO, S.** ; **JEONG, J.** ; **GUNAWIDJAJA, R.** ; **TSUKRUK, V. V.** Amphiphilic Heteroarm PEO-b-PSm Star Polymers at the Air-Water Interface: Aggregation and Surface Morphology.. *Macromolecules*, 2004, vol. 37, 6511-6522
- **CHEYNE, R. B.** ; **MOFFITT, M. G.** Self-Assembly of Polystyrene-block-Poly(Ethylene Oxide) Copolymers at the Air-Water Interface: Is Dewetting the Genesis of Surface Aggregate Formation?. *Langmuir*, 2006, vol. 22, 8387-8396
- **LEVITT, M. H.** Spin Dynamics: Basics of Nuclear Magnetic Resonance. Wiley, 2001
- **RIES, M. E.** ; **KLEIN, P. G.** ; **BRERETON, M. G.** ; **WARD, I. M.** Proton NMR Study of Rouse Dynamics and Ideal Glass Transition Temperature of Poly(ethylene oxide) LiCF3SO3 Complexes.. *Macromolecules*, 1998, vol. 31, 4950-4956
- **ZHENG, Y. et al.** Directly Resolved Core-Corona Structure of Block Copolymer Micelles by Cryo-Transmission Electron Microscopy.. *The Journal of Physical Chemistry B*, 1999, vol. 103, 10331-10334
- **WON, Y.-Y.** ; **DAVIS, H. T.** ; **BATES, F. S.** ; **AGAMALIAN, M.** ; **WIGNALL, G. D.** Segment Distribution of the Micellar Brushes of Poly(ethylene oxide) via Small-Angle Neutron Scattering.. *The Journal of Physical Chemistry B*, 2000, vol. 104, 7134-7143
- **NAKOS, G.** ; **KITSIOULI, E. I.** ; **TSANGARIS, I.** ; **LEKKA, M. E.** Bronchoalveolar lavage fluid characteristics of early intermediate and late phases of ARDS.. *Intensive Care Medicine*, 1998, vol. 24, 296-303
- **SPRAGG, R. G.** Pathophysiology of Shock, Sepsis, and Organ Failure. Springer, 1993, 747-756
- **SPRAGG , R. G. et al.** Effect of Recombinant Surfactant Protein C-Based Surfactant on the Acute Respiratory Distress Syndrome.. *New England Journal of Medicine*, 2004, vol. 351, 884-892
- **GREGORY, T. J. et al.** Bovine surfactant therapy for patients with acute respiratory distress syndrome.. *American Journal of Respiratory and Critical Care Medicine*, 1997, vol. 155, 1309-1315
- **FILOCHE, M.** ; **TAI, C.-F.** ; **GROTBERG, J. B.** Three-dimensional model of surfactant replacement therapy.. *Proceedings of the National Academy of Sciences*, 2015, vol. 112, 9287-9292
- **GROTBERG, J. B.** ; **FILOCHE, M.** ; **WILLSON, D. F.** ; **RAGHAVENDRAN, K.** ; **NOTTER, R. H.** Did Reduced Alveolar Delivery of Surfactant Contribute to Negative Results in Adults with Acute Respiratory Distress Syndrome?. *American Journal of Respiratory and Critical Care Medicine*, 2017, vol. 195, 538-540
- **KING, D. M.** ; **WANG, Z.** ; **PALMER, H. J.** ; **HOLM, B. A.** ; **NOTTER, R. H.** Bulk shear viscosities of endogenous and exogenous lung surfactants.. *American Journal of Physiology - Lung Cellular and Molecular Physiology*, 2002, vol. 282, L277-L284
- **KING, D. M. et al.** Concentration-dependent, temperature-dependent non-Newtonian viscosity of lung surfactant dispersions.. *Chemistry and Physics of Lipids*, 2001, vol. 112, 11-19
- **BERGGREN, E. et al.** Pilot study of nebulized surfactant therapy for neonatal respiratory distress syndrome.. *Acta Pædiatrica*, 2000, vol. 89, 460-464
- **LEWIS, J. F.** ; **IKEGAMI, M.** ; **JOBE, A. H.** ; **TABOR, B.** Aerosolized surfactant treatment of preterm lambs.. *Journal of Applied Physiology*, 1991, vol. 70, 869-876
- **ELLYETT, K. M.** ; **BROADBENT, R. S.** ; **FAWCETT, E. R.** ; **CAMPBELL, A. J.** Surfactant Aerosol Treatment of Respiratory Distress Syndrome in the Spontaneously Breathing Premature Rabbit.. *Pediatr Res*, 1996, vol. 39, 953-957
- **DIJK, P. H.** ; **HEIKAMP, A.** ; **OETOMO, S. B.** Surfactant Nebulization versus Instillation during High Frequency Ventilation in Surfactant-Deficient Rabbits.. *Pediatr Res*, 1998, vol. 44, 699-704
- **EDWARDS, D. A.** ; **BEN-JEBRIA, A.** ; **LANGER, R.** Recent advances in pulmonary drug delivery using large, porous inhaled particles. *Journal of Applied Physiology*, 1998, vol. 85, 379-385

- **SUNG, J. C.** ; **PULLIAM, B. L.** ; **EDWARDS, D. A.** Nanoparticles for drug delivery to the lungs. *Trends in Biotechnology*, 2007, vol. 25, 563-570, http://dx.doi.org/10.1016/j.tibtech.2007.09.005
- **TSAPIS, N.** ; **BENNETT, D.** ; **JACKSON, B.** ; **WEITZ, D. A.** ; **EDWARDS, D. A.** Trojan particles: Large porous carriers of nanoparticles for drug delivery.. *Proceedings of the National Academy of Sciences*, 2002, vol. 99, 12001-12005
- **UNGARO, F. et al.** Dry powders based on PLGA nanoparticles for pulmonary delivery of antibiotics: Modulation of encapsulation efficiency, release rate and lung deposition pattern by hydrophilic polymers.. *Journal of Controlled Release*, 2012, vol. 157, 149-159, http://dx doi.org/10.1016/j.jconrel.2011.08.010
- **YAMAMOTO, H.** ; **KUNO, Y.** ; **SUGIMOTO, S.** ; **TAKEUCHI, H.** ; **KAWASHIMA, Y.** Surface-modified PLGA nanosphere with chitosan improved pulmonary delivery of calcitonin by mucoadhesion and opening of the intercellular tight junctions.. *Journal of Controlled Release*, 2005, vol. 102, 373-381, http://dx.doi.org/10.1016/j.jconrel.2004.10.010
- **MENON, J. U. et al.** Polymeric Nanoparticles for Pulmonary Protein and DNA Delivery. *Acta biomaterialia*, 2014, vol. 10, 2643-2652
- **NEISES, B.** ; **STEGLICH, W.** Simple Method for the Esterification of Carboxylic Acids.. *Angewandte Chemie International Edition in English*, 1978, vol. 17, 522-524